(19) Europäisches Patentamt
European Patent Office
Office européen des brevets

(11) Veröffentlichungsnummer: **0 068 240**
**B1**

(12) # EUROPÄISCHE PATENTSCHRIFT

(45) Veröffentlichungstag der Patentschrift: **25.09.85**

(21) Anmeldenummer: **82105139.8**

(22) Anmeldetag: **12.06.82**

(51) Int. Cl.⁴: **C 07 D 405/14**, C 07 D 409/14, C 07 D 409/04, C 07 D 405/04, C 07 D 401/04, C 07 D 403/04, A 61 K 31/55

(54) **2-Acylaminomethyl-1,4-benzodiazepin-Verbindungen sowie Verfahren und Zwischenprodukte zu ihrer Herstellung und diese Verbindungen enthaltende Arzneimittel.**

(30) Priorität: **19.06.81 DE 3124013**

(43) Veröffentlichungstag der Anmeldung: **05.01.83 Patentblatt 83/1**

(45) Bekanntmachung des Hinweises auf die Patenterteilung: **25.09.85 Patentblatt 85/39**

(84) Benannte Vertragsstaaten: **AT BE CH DE FR GB IT LI LU NL SE**

(56) Entgegenhaltungen:
**EP - A - 0 031 080**
**DE - A - 2 314 993**
**DE - A - 2 353 160**
**DE - A - 2 353 187**
**DE - A - 2 436 147**

(73) Patentinhaber: **Kali-Chemie Pharma GmbH, Hans-Böckler-Allee 20 Postfach 220, D-3000 Hannover 1 (DE)**

(72) Erfinder: **Zeugner, Horst, Dipl.-Chem. Dr.rer.nat., Havelweg 10, D-3000 Hannover 73 (DE)**
Erfinder: **Römer, Dietmar, Dr., Lettenweg 111, CH-4123 Allschwil (CH)**
Erfinder: **Benson, Werner, Dipl.-Chem. Dr.rer.nat., In der Steinbreite 39, D-3000 Hannover 91 (DE)**
Erfinder: **Liepmann, Hans, Dipl.-Chem.Dr.rer.nat., Auf dem Emmerberg 17, D-3000 Hannover 1 (DE)**
Erfinder: **Milkowski, Wolfgang, Dipl.-Chem.Dr.rer.nat., Fasanenweg 13, D-3167 Burgdorf (DE)**

(74) Vertreter: **Lauer, Dieter, Dr., c/o Kali-Chemie AG Postfach 220 Hans-Böckler-Allee 20, D-3000 Hannover 1 (DE)**

EP 0 068 240 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-Acylaminomethyl -5- heteroaryl -1H-2,3- dihydro -1,4- benzodiazepin-Verbindungen und deren Salze sowie diese Verbindungen enthaltende pharmazeutische Zubereitungen und Verfahren und Zwischenprodukte zur Herstellung dieser Verbindungen.

Aus der DE-OS 2 353 187 sind u.a. 2-Acylaminomethyl-1,4- benzodiazepine beschrieben, in welchen der Acylrest ein niedermolekulares Alkanoyl ist. Diese Substanzen besitzen vor allem eine antikonvulsive Wirkung.

Der Erfindung liegt die Aufgabe zugrunde, neue 2-Acylaminomethyl-1,4-benzodiazepin-Verbindungen mit wertvollen pharmakologischen Eigenschaften zu entwickeln.

Es wurde nun gefunden, dass die neuen 2-Acylaminomethyl -5- heteroaryl -1,4- benzodiazepin-Verbindungen wertvolle pharmakologische Wirkungen, insbesondere analgetische Wirkungen, besitzen und ein vorteilhaftes Wirkungsprofil mit guter therapeutischer Breite und geringer Toxizität aufweisen.

Die vorliegende Erfindung betrifft daher neue 2-Acylaminomethyl-1H -2,3- dihydro -1,4- benzodiazepin-Verbindungen der allgemeinen Formel I

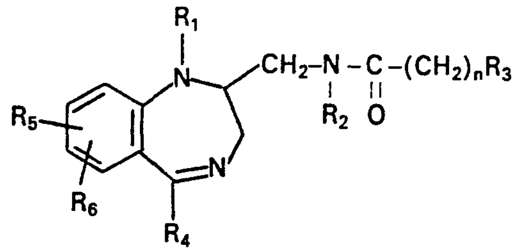

I

worin

$R_1$ Wasserstoff, Alkyl mit 1–4 Kohlenstoffatomen, Allyl, 2-Butenyl, 3-Butenyl oder Cyclopropylmethyl bedeutet,

$R_2$ Wasserstoff, Alkyl mit 1–4 Kohlenstoffatomen, Allyl, 2-Butenyl oder 3-Butenyl bedeutet,

n für 0, 1 oder 2 steht,

$R_3$ für eine der folgenden Gruppen a, b, c oder d steht

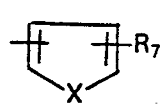

a

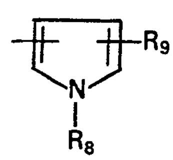

b

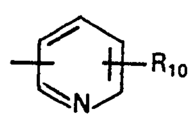

c

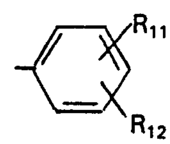

d

worin

X Sauerstoff oder Schwefel bedeutet,

$R_7$ Wasserstoff, Alkyl mit 1–4 Kohlenstoffatomen, Alkoxy mit 1–4 Kohlenstoffatomen, Nitro oder Halogen bedeutet,

$R_8$ Wasserstoff oder Alkyl mit 1–4 Kohlenstoffatomen bedeutet,

$R_9$ Wasserstoff oder Alkyl mit 1–4 Kohlenstoffatomen bedeutet,

$R_{10}$ Wasserstoff, Alkyl mit 1–4 Kohlenstoffatomen oder Chlor bedeutet,

$R_{11}$ Wasserstoff, Alkyl mit 1–4 Kohlenstoffatomen, Alkoxy mit 1–4 Kohlenstoffatomen, Hydroxy, Halogen, Alkylthio mit 1–4 Kohlenstoffatomen, Nitro, Trifluormethyl, Cyano, Amino, durch Alkyl mit 1 – 4 Kohlenstoffatomen mono- oder disubstituiertes Amino, Monoalkanoylamino mit 1–4 Kohlenstoffatomen, N-$C_1$–$C_4$-Alkyl–N-$C_1$–$C_4$ -alkanoylamino oder Alkanoyloxy mit 1–4 Kohlenstoffatomen bedeutet,

$R_{12}$ Wasserstoff, Alkyl mit 1–4 Kohlenstoffatomen, Alkoxy mit 1–4 Kohlenstoffatomen oder Halogen bedeutet, oder

$R_{11}$ und $R_{12}$ an benachbarte Kohlenstoffatome gebunden sind und zusammen Methylendioxy oder Äthylendioxy bedeuten,

$R_4$ für eine der vorgehend definierten Gruppen a, b oder c steht,

$R_5$ Wasserstoff, Alkyl mit 1–4 Kohlenstoffatomen, Alkoxy mit 1–4 Kohlenstoffatomen, Hydroxy, Halogen, Alkylthio mit 1–4 Kohlenstoffatomen, Nitro, Trifluormethyl, Cyano, Amino, durch Alkyl mit 1–4 Kohlenstoffatomen mono- oder disubstituiertes Amino, Monoalkanoylamino mit 1– 4 Kohlenstoffatomen, N-$C_1$ –$C_4$ –Alkyl–N-$C_1$ –$C_4$ -alkanoylamino oder Alkanoyloxy mit 1–4 Kohlenstoffatomen bedeutet, und

$R_6$ Wasserstoff, Alkyl mit 1–4 Kohlenstoffatomen, Alkoxy mit 1–4 Kohlenstoffatomen oder Halogen bedeutet, oder

$R_5$ und $R_6$ an benachbarte Kohlenstoffatome gebunden sind und zusammen Methylendioxy oder Äthylendioxy bedeuten, sowie deren optische Isomere und deren Säureadditionssalze.

Sofern in den Verbindungen der Formel I die Substituenten $R_5$ und $R_6$ des Phenylringes oder die in den Resten $R_3$ und $R_4$ enthaltenen Substituenten $R_7$ bis $R_{12}$ eine niedere Alkylgruppe enthalten, kann diese gerade oder verzweigt sein und 1–4 Kohlenstoffatome enthalten. So kommen insbesondere Methyl, Äthyl, n-Propyl, Isopropyl, n-Butyl, 1-Methylpropyl, 2-Methylpropyl oder tert. Butyl, bevorzugt Methyl, Äthyl, n-Propyl und Isopropyl, in Frage. Insbesondere bei Di- und Trisubstitution an den Phenylringen sind Äthyl und insbesondere Methyl bevorzugt. Niedermolekulare Alkoxy- oder Alkylthiosubstituenten stellen vorzugsweise Methoxy oder Methylthio dar.

Als Alkyl- und Alkenylgruppen $R_1$ und $R_2$ kommen niedermolekulare Gruppen mit bis zu 4 Kohlenstoffatomen in Frage, die gerade oder verzweigt sind, z.B. Methyl, Äthyl, Propyl, Isopropyl,

n-Butyl, 1-Methylpropyl, 2-Methylpropyl, tert. Butyl, Allyl, 2-Butenyl oder 3-Butenyl. Der Substituent $R_1$ steht insbesondere für Wasserstoff, niederes Alkyl oder Cyclopropylmethyl, vorzugsweise für niederes Alkyl, insbesondere Methyl. $R_2$ stellt bevorzugt Wasserstoff dar.

Für die Substituenten $R_5$ und/oder $R_6$ kommen als Halogenatome insbesondere Fluor, Chlor oder Brom in Frage. Die Substituenten $R_5$ und $R_6$ sind bevorzugt in 7- und/oder 8-Stellung angeordnet. Falls $R_5$ für Alkylthio, Nitro, Trifluormethyl, Cyano oder gegebenenfalls substituiertes Amino steht, ist $R_6$ vorzugsweise Wasserstoff. Im Falle von Halogen und/oder Alkyl bzw. und/oder Alkoxy-Substituenten ist Mono- und Disubstition zweckmässig.

Sofern $R_3$ für eine gegebenenfalls substituierte Phenylgruppe d steht, trifft für die Substituenten $R_{11}$ und $R_{12}$ das vorstehend für die Substituenten $R_5$ und $R_6$ Gesagte ebenfalls zu.

Falls $R_3$ und/oder $R_4$ für eine Gruppe a, worin X Sauerstoff bedeutet, stehen, bedeutet $R_7$ insbesondere Wasserstoff, niederes Alkyl oder Nitro, bevorzugt Wasserstoff oder Methyl. Falls $R_3$ und/oder $R_4$ für eine Gruppe a, worin X Schwefel bedeutet, stehen, steht $R_7$ insbesondere für Wasserstoff, niederes Alkyl, bevorzugt Methyl, Halogen, insbesondere Chlor oder Brom, oder auch Nitro oder niederes Alkoxy.

Falls $R_3$ und/oder $R_4$ für eine Pyrrolylgruppe b stehen, bedeutet $R_8$ insbesondere Wasserstoff oder Methyl und $R_9$ insbesondere Wasserstoff oder Methyl.

Die Gruppen a – c können in 2- oder 3-Stellung, zweckmässigerweise in 3-Stellung, mit dem Benzodiazepin-Gerüst verbunden sein.

Falls $R_3$ und/oder $R_4$ für eine Pyridylgruppe c stehen, bedeutet $R_{10}$ insbesondere Wasserstoff. Falls $R_{10}$ niederes Alkyl bedeutet, kommt bevorzugt Methyl in Frage.

Erfindungsgemäss werden die neuen 2-Acylaminomethyl- 1H-2,3- dihydro-1,4-benzodiazepin-Verbindungen der Formel I sowie deren optische Isomere und Säureadditionssalze erhalten, indem man in an sich bekannter Weise 2-Aminomethyl-1H-2,3-dihydro-1,4-benzodiazepin-Verbindungen der Formel II

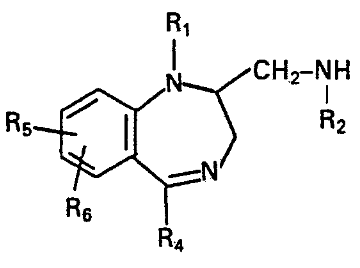

II

worin $R_1$, $R_2$, $R_4$, $R_5$ und $R_6$ obige Bedeutung besitzen, mit Säuren oder reaktionsfähigen Säurederivaten der Formel III

$R_3$–$(CH_2)_n$–CO–Y III

worin $R_3$ und n obige Bedeutung besitzen und Y Hydroxy oder eine aminolytisch abspaltbare Gruppe bedeutet, acyliert und gegebenenfalls Verbindungen der Formel I, worin $R_2$ Wasserstoff bedeutet, zu Verbindungen der Formel I, worin $R_2$ niederes Alkyl oder Alkenyl bedeutet, alkyliert und gegebenenfalls racemische Gemische von Verbindungen der Formel I in ihre optischen Isomeren auftrennt und gegebenenfalls freie Verbindungen der Formel I in ihre Säureadditionssalze überführt oder die Säureadditionssalze in die freien Verbindungen der Formel I überführt.

Die Acylierung der Aminomethyl-Verbindungen der Formel II kann nach an sich zur Bildung von Amid-Gruppierungen durch Aminoacylierung üblichen Methoden ausgeführt werden. Als Acylierungsmittel können Säuren der Formel IIIa

$R_3$–$(CH_2)_n$–CO–OH IIIa

worin $R_3$ und n obige Bedeutung besitzen, oder deren reaktionsfähige Derivate eingesetzt werden. Als reaktionsfähige Derivate kommen insbesondere Säurehalogenide, vorzugsweise -chloride, Säureester und -anhydride in Frage, z.B. Verbindungen der Formel III, worin der aminolytisch abspaltbare Rest Y Halogen, insbesondere Chlor oder Brom, niederes Alkoxy, insbesondere Alkoxy mit 1 bis 4 Kohlenstoffatomen, oder eine Gruppe O–CO–Z bedeutet, worin Z für die $R_3(CH_2)_n$-Gruppe oder für niederes Alkyl oder Alkoxy steht. Die Acylierung kann in einem unter den Reaktionsbedingungen inerten Lösungsmittel bei Temperaturen zwischen –30 °C und dem Siedepunkt des Lösungsmittels bei Normaldruck oder bei erhöhtem Druck erfolgen. Als Lösungsmittel eignen sich halogenierte Kohlenwasserstoffe wie Methylenchlorid oder Chloroform, aromatische Kohlenwasserstoffe wie Benzol, Toluol, Xylol oder Chlorbenzol, cyclische Äther wie Tetrahydrofuran oder Dioxan, Ketone wie z.B. Aceton oder Methylisobutylketon, oder Dimethylformamid oder Gemische dieser Lösungsmittel.

Die Acylierung kann gegebenenfalls, insbesondere wenn als Acylierungsmittel ein Carbonsäurehalogenid oder -anhydrid verwendet wird, in Gegenwart eines säurebindenden Reagenzes durchgeführt werden. Als säurebindende Mittel eignen sich anorganische Basen, insbesondere Alkalimetallcarbonate und -hydroxide wie z.B. Natrium- oder Kaliumcarbonat oder Kaliumhydroxid, oder organische Basen, insbesondere tertiäre Niederalkylamine und Pyridine wie z.B. Triäthylamin, Tripropylamin, Tributylamin, Pyridin, 4-Dimethylaminopyridin oder 4-Pyrrolidinopyridin. Im Überschuss eingesetzte organische Basen können gleichzeitig auch als Lösungsmittel dienen.

Falls als Acylierungsmittel die Säure selbst oder auch ein Ester eingesetzt wird, so wird die Umsetzung der Aminoverbindung der Formel II mit der Säure der Formel IIIa oder auch deren Ester zweckmässigerweise in Gegenwart eines aus der Peptidchemie als zur Amidbildung geeignet bekannten Kopplungsgreagenzes durchgeführt. Als Beispiele von Kopplungsreagenzien, welche die Amidbildung mit den freien Säuren dadurch för-

dern, dass sie mit der Säure in situ unter Bildung eines reaktionsfähigen Säurederivates reagieren, seien insbesondere genannt Alkyl-, vorzugsweise Cycloalkylcarbodiimide wie Dicyclohexylcarbodiimid, Carbonyldiimidazol und N-niederalkyl-2-halogenpyridiniumsalze, insbesondere Halogenide oder Tosylate, vorzugsweise N-Methyl-2-chlorpyridiniumjodid (siehe z.B. Mukaiyama in Angew. Chemie 91 789–812). Die Umsetzung in Gegenwart eines Kopplungsreagenzes kann zweckmässig bei Temperaturen von –30 bis +30 °C unter Benutzung von Lösungsmitteln wie halogenierten Kohlenwasserstoffen und/oder aromatischen Lösungsmitteln gegebenenfalls in Gegenwart eines säurebindenden Amins durchgeführt werden. Als Beispiele von Kopplungsreagenzien, welche die Amidbildung mit den Estern durch Bildung eines reaktionsfähigen Derivates der Aminoverbindung fördern, seien genannt Triniederalkylaluminium, insbesondere Trimethylaluminium, welches zur Aktivierung der Umsetzung der Aminoverbindungen mit Estern geeignet ist, oder $PCl_3$. Als inerte Lösungsmittel für die Umsetzung in Gegenwart von Trialkylaluminium eignen sich insbesondere aromatische Kohlenwasserstoffe und/oder halogenierte Kohlenwasserstoffe. Die Umsetzung der Aminoverbindung mit dem Trialkylaluminium wird vorzugsweise bei Temperaturen von –20 °C bis Raumtemperatur durchgeführt, die anschliessende Reaktion der intermediär gebildeten Monoalkylaluminiumazoverbindungen mit dem Ester kann insbesondere bei Temperaturen zwischen Raumtemperatur und Siedepunkt des Lösungsmittels durchgeführt werden. Weitere zur erfindungsgemässen Amidbildung geeignete Kopplungsreagenzien, welche auch in Peptidsynthesen Anwendung finden, sind z.B. bekannt aus Advanced Organic Chemistry by Jerry March McGraw-Hill Ltd., 2. Ausgabe, Seite 382 bis 388, und aus The Chemistry of Amides by Jacob Zabicky 1970 Interscience Publishers John Wiley and Sons, London Chapter 2: Synthesis of Amides.

Falls die Substituenten $R_3$, $R_4$, $R_5$ und/oder $R_6$ freie Hydroxy- oder Aminogruppen enthalten, können diese gewünschtenfalls vor der Acylierung mit einer Schutzgruppe versehen werden, welche nach der Umsetzung wieder abspaltbar, beispielsweise hydrolytisch abspaltbar ist. Die freien Amino-, Monoalkylamino- und Hydroxygruppen lassen sich z.B. als leicht wieder abspaltbare Sulfinylimino-, Acetylalkylamino- oder Acetoxygruppen schützen. Falls freie ungeschützte phenolische Hydroxylgruppen vorhanden sind, können diese bei der Acylierung teilweise mitacyliert werden, allfällige dadurch gebildete Phenolestergruppen sind jedoch leicht und selektiv hydrolytisch spaltbar, beispielsweise durch Behandlung mit Natriumcarbonatlösung.

Falls $R_2$ und/oder $R_4$ einen Pyrrolring darstellen, ist es zweckmässig, die Acyllierung unter schonenden Reaktionsbedingungen, beispielsweise unter Verwendung eines der vorstehend genannten Kopplungsreagenzien durchzuführen.

Erhaltene Verbindungen der Formel I, worin $R_2$ Wasserstoff bedeutet, können gewünschtenfalls nachträglich in an sich bekannter Weise zu den entsprechenden N-Alkyl-Verbindungen alkyliert werden. Als Alkylierungsmittel kommen Alkylhalogenide, Alkylsulfate oder Alkylsulfonsäureester in Frage. Zweckmässigerweise wird die Amido-Verbindung der Formel II zunächst mit einem Metallierungsmittel wie beispielsweise einem Alkalimetallhydrid, -amid oder -alkoholat oder einer lithiumorganischen Verbindung oder einem Thallium-I-alkoholat in einem inerten Lösungsmittel umgesetzt und anschliessend die metallierte Verbindung weiter mit dem Alkylierungsmittel umgesetzt. Die Umsetzung kann bei einer Temperatur von –80 °C bis zur Siedetemperatur des Lösungsmittels erfolgen. Als Metallierungsmittel eignen sich insbesondere Natriumhydrid, Lithiumbutyl, Lithiumphenyl, Natriumamid, Lithiumdiisopropylamid, Natriumalkoxide oder Thallium-I-alkoxide. Je nach dem verwendeten Metallierungsmittel eignen sich als Lösungsmittel offene und cyclische Äther wie Diäthyläther, Tetrahydrofuran oder Dioxan, aromatische Kohlenwasserstoffe wie Benzol oder Toluol, Dimethylformamid, Dimethylsulfoxid oder, falls das Metallierungsmittel ein Metallalkoholat ist, auch die entsprechenden Alkohole, also Methanol im Falle von Methoxiden und Äthanol im Falle von Äthoxiden.

Die erfindungsgemässen Verbindungen der allgemeinen Formel I werden bei der Synthese in Form ihrer Racemate erhalten. Die vorliegende Erfindung umfasst nun sowohl die racemischen Gemische wie auch die optisch aktiven Formen der Verbindungen der Formel I. Die optisch aktiven Verbindungen können aus den racemischen Gemischen in an sich bekannter Weise durch Umsetzung mit geeigneten optisch aktiven Säuren, wie beispielsweise Weinsäure, O,O'-Dibenzoylweinsäure, Mandelsäure, Di-O-Isopropyliden-2-oxo-L-gulonsäure, und anschliessende fraktionierte Kristallisation der gewonnenen Salze in ihre optisch aktiven Antipoden aufgetrennt werden (siehe z.B. Tetrahedron 33 (1977) Seiten 2725–2736).

Die Auftrennung in die optisch aktiven Verbindungen kann gewünschtenfalls auch in einer geeigneten Vorstufe erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Verbindungen der allgemeinen Formel IIa

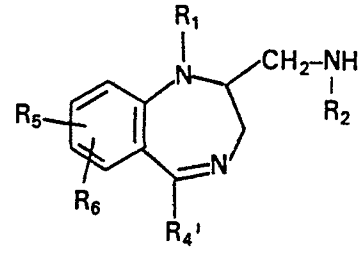

IIa

worin $R_1$, $R_2$, $R_5$, $R_6$ obige Bedeutung zu setzen und $R_4'$ für eine der folgenden Gruppen a', b oder c steht

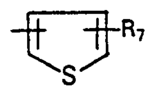
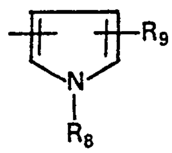
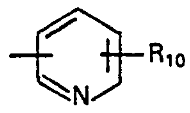

a' b c

worin $R_7$, $R_8$, $R_9$ und $R_{10}$ obige Bedeutung besitzen, stellen neue wertvolle Zwischenprodukte für die Herstellung von pharmakologisch aktiven Verbindungen, beispielsweise den Verbindungen der Formel I, dar. Verbindungen der Formel II können erhalten werden, indem man in Verbindungen der Formel IV

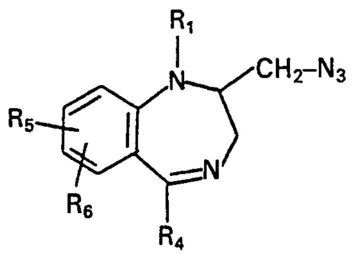

IV

worin $R_1$, $R_5$, $R_6$ und $R_4$ obige Bedeutung besitzen, die Azidogruppe zur Aminogruppe reduziert oder in Verbindungen der Formel V

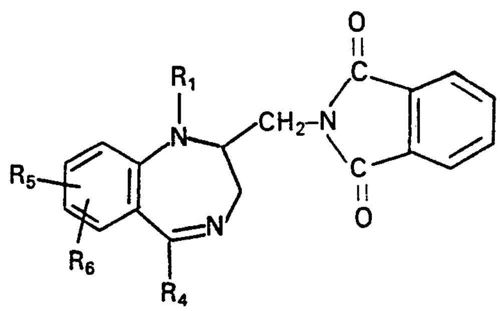

V

worin $R_1$, $R_5$, $R_6$ und $R_4$ obige Bedeutung besitzen, die Phthalimidgruppe zur Aminogruppe hydrolisiert und die erhaltenen Verbindungen der Formel IIb

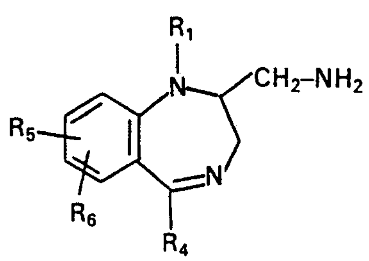

IIb

worin $R_1$, $R_5$, $R_6$ und $R_4$ obige Bedeutung besitzen, gewünschtenfalls in an sich bekannter Weise alkyliert.

Die reduktive Spaltung von Verbindungen der Formel IV kann auf an sich bekannte Weise mit zur Reduktion von Aziden bekannten Reduktionsmitteln durchgeführt werden.

Als Reduktionsmittel eignen sich beispielsweise Hydrazinhydrat/Raney-Nickel, 1,3-Dimercaptopropan, oder Zinn-II-chlorid. Die Reduktion mit Hydrazinhydrat/Raney-Nickel erfolgt zweckmässigerweise in einem inerten Lösungsmittel, beispielsweise in einem niederen Alkohol, unter basischer Katalyse, beispielsweise unter Zusatz eines tertiären Amines, z.B. eines nieder-Alkylamins wie Triäthylamin, vorzugsweise bei Raumtemperatur. Die Reduktion mit Dimercaptopropan erfolgt ebenfalls in einem inerten Lösungsmittel, gegebenenfalls unter basischer Katalyse. Hierbei eignen sich als Lösungsmittel beispielsweise niedere Alkohole, Dimethylformamid oder Pyridin/Wasser. Die Reduktion mit Zinn-II-chlorid erfolgt zweckmässigerweise in konzentrierter Salzsäure.

Die Hydrolyse der Phthalimidverbindungen der Formel V kann auch auf an sich bekannte Weise sauer, beispielsweise durch Zusatz von verdünnter Salzsäure, oder alkalisch, beispielsweise durch Zusatz von Hydrazinhydrat, in Wasser oder einem Gemisch aus Wasser und einem wasserlöslichen organischen Lösungsmittel, vorzugsweise einem niederen Alkohol, bei Temperaturen zwischen 20–120 °C, vorzugsweise Siedetemperatur des Reaktionsgemisches, erfolgen.

Die Alkylierung von Verbindungen der Formel IIb kann nach an sich zur Aminoalkylierung üblichen Methoden, beispielsweise durch Umsetzung mit Alkylhalogeniden, Alkylsulfaten oder Alkylsulfonsäureestern oder durch reduktive Alkylierung erfolgen.

Die Verbindungen der Formel II können auch erhalten werden, indem man Verbindungen der Formel IX und/oder Verbindungen der Formel X

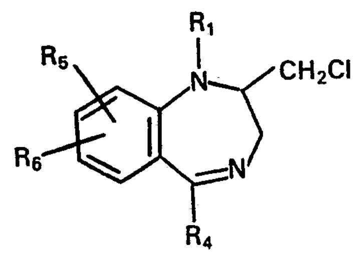

IX

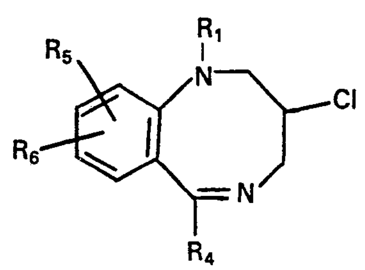

X

worin $R_1$, $R_5$, $R_6$ und $R_4$ obige Bedeutung besitzen, mit einem Amin der Formel XI

$R_2NH_2$ XI

worin $R_2$ obige Bedeutung besitzt, umsetzt.

Die Umsetzung der Verbindungen der Formel IX und/oder X, vorzugsweise eines Gemisches der isomeren Verbindungen IX und X, mit Ammoniak oder einem primären nieder-Alkylamin, wie beispielsweise Methylamin, Äthylamin, Propylamin,

Butylamin oder Allylamin, erfolgt in an sich bekannter Weise bei Temperaturen zwischen 20 und 150 °C, vorzugsweise Siedetemperatur des Reaktionsgemisches. Ein Überschuss des Amins kann als Lösungsmittel dienen. Gewünschtenfalls kann auch ein inertes Lösungsmittel zugesetzt werden, beispielsweise ein niederer Alkohol wie Methanol, Äthanol, Isopropanol oder tert.Butanol, ein cyclischer Äther wie Dioxan oder ein aromatischer Kohlenwasserstoff wie Benzol, Toluol oder Xylol. Gewünschtenfalls können an Stelle von Ammoniak oder dem primären Amin deren Alkalimetallsalze mit den Verbindungen der Formel IX und/oder X umgesetzt werden. Als Lösungsmittel dient in diesem Falle vorzugsweise ein Überschuss des jeweiligen Amins oder flüssiger Ammoniak. Es können aber auch andere inerte Lösungsmittel, beispielsweise cyclische Äther oder aromatische Kohlenwasserstoffe zugesetzt werden. Die Alkalimetallsalze der Amine können in situ erzeugt oder in fester Form zugesetzt werden. Die Temperaturen für die Umsetzungen können zwischen –50 und +150 °C liegen.

Die Verbindungen der Formel IV sind in der Literatur bisher noch nicht beschrieben worden. Sie stellen neue, wertvolle Zwischenprodukte zur Herstellung von pharmakologisch aktiven Verbindungen, beispielsweise den Verbindungen der Formel I, dar. Darüber hinaus weisen sie auch selbst wertvolle pharmakologische Wirkungen auf. Insbesondere zeigen sie für Sedativa und Psychopharmaka charakteristische Eigenschaften.

Erfindungsgemäss können die Verbindungen der Formel IV erhalten werden, indem man Verbindungen der Formel IX und/oder Verbindungen der Formel X mit einem Alkalimetallazid umsetzt.

Die Umsetzung erfolgt nach zur Azidbildung üblichen Methoden. Beispielsweise werden die Verbindungen der Formel IX und/oder X, insbesondere ein Gemisch der isomeren Verbindungen der Formel IX und X, mit einem Alkalimetallazid vorzugsweise Natrium- oder Kaliumazid in einem inerten Lösungsmittel bei Temperaturen von –30 – 150 °C umgesetzt. Geeignete Lösungsmittel sind beispielsweise halogenierte Kohlenwasserstoffe wie Methylenchlorid oder Chloroform, cyclische Äther wie Tetrahydrofuran oder Dioxan, Dimethylformamid, Dimethylsulfoxid, Hexamethylphosphortriamid, niedere Alkohole, wie Methanol, Äthanol oder tert. Butanol, niedere Ketone wie Aceton oder Methylisopropylketon.

Die Verbindungen der Formel IV können auf an sich bekannte Weise aus dem Reaktionsgemisch isoliert und gereinigt werden. Sie können aber auch gegebenenfalls nach Entfernung des Lösungsmittels ohne weitere Reinigung weiter umgesetzt werden. Die freien Verbindungen der Formel IV lassen sich in üblicher Weise in ihre Säureadditionssalze überführen und umgekehrt.

Gewünschtenfalls können racemische Gemische von Verbindungen der Formel IV in an sich bekannter Weise in ihre optischen Isomeren aufgetrennt werden.

Verbindungen der Formel V können erhalten werden, indem man Verbindungen der Formel IX und/oder Verbindungen der Formel X mit einem Alkalimethallphthalimid umsetzt. Die Umsetzung erfolgt zweckmässigerweise in einem inerten Lösungsmittel, beispielsweise einem niederen Alkohol wie Methanol, Äthanol oder Isopropanol, einem cyclischen Äther wie Dioxan oder Dimethylformamid bei Temperaturen zwischen 50 und 130 °C. Gewünschtenfalls kann Kaliumjodid als Katalysator zugegeben werden.

Die Herstellung der als Ausgangssubstanzen verwendeten Verbindungen der Formel IX und X kann in an sich bekannter Weise erfolgen. Beispielsweise können Verbindungen, worin $R_4$ eine Gruppe a bedeutet, nach den in den Deutschen Offenlegungsschriften Nr. 2 221 558 oder 2 353 187 beschriebenen Methoden erhalten werden.

Insbesondere kann man 2-Hydroxy -1,3- diaminopropane der allgemeinen Formel VI

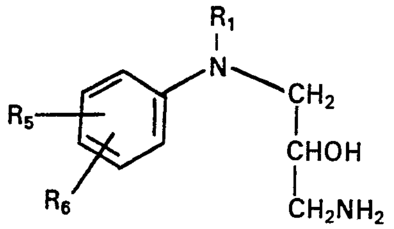

VI

worin $R_1$, $R_5$ und $R_6$ obige Bedeutung besitzen, mit einem Acylchlorid der Formel VII

$R_4''$–CO–Cl VII

worin $R_4''$ eine Gruppe a bedeutet, zu Acylaminen der allgemeinen Formel VIII

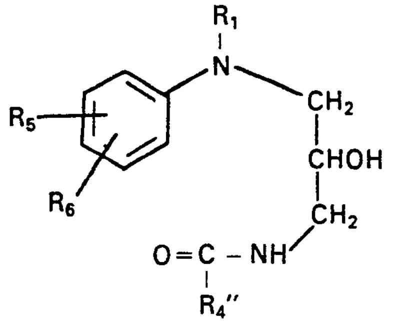

VIII

worin $R_1$, $R_4''$, $R_5$ und $R_6$ obige Bedeutung besitzen, acyllieren und anschliessend die Verbindungen der Formel VIII in an sich bekannter Weise durch Umsetzung mit Phosphoroxidhalogeniden, vorzugsweise Phosphoroxidchlorid, cyclisieren. Zweckmässigerweise behandelt man dazu die Verbindungen der Formel VIII oder deren Säureadditionssalze wie in der Deutschen Offenlegungsschrift Nr. 2 520 937 beschrieben mit dem Phosphoroxidchlorid bei einer Temperatur zwischen 100 und 150 °C, vorzugsweise bei der Siedetemperatur des Reaktionsgemisches. Hierbei wird ein Gemisch der beiden isomeren Verbindungen IX und X gebildet, welches auf an sich bekannte Weise aus dem Reaktionsgemisch isoliert werden kann.

Die beiden isomeren Verbindungen IX und X liegen in dem isomeren Gemisch in wechselnden Mengenverhältnissen vor in Abhängigkeit von der Art der Substituenten $R_4''$, $R_5$ und $R_6$. Dies ist jedoch für die folgende Umsetzung dieses Gemisches ohne Belang, da beide Isomere in einheitlicher Reaktion zu Verbindungen der allgemeinen Formel II bzw. Verbindungen der allgemeinen Formeln IV und V reagieren. Eine langwierige Trennung oder Analyse des Isomerengemisches ist daher vor der weiteren Umsetzung nicht nötig. Selbstverständlich aber können gewünschtenfalls auch die Isomeren auf dieser Stufe getrennt und einzeln für die folgenden Umsetzungen eingesetzt werden.

Zur Herstellung von Verbindungen der Formel IX, worin $R_4$ eine Gruppe a bedeutet, kann man auch ausgehen von 2-Aminobenzoyl-Verbindungen der Formel XII

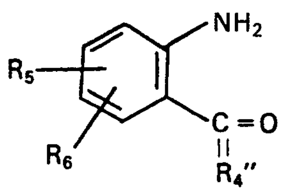

XII

worin $R_4''$, $R_5$ und $R_6$ obige Bedeutung besitzen. Diese werden zunächst mit Epichlorhydrin zu Verbindungen der Formel XIIIa

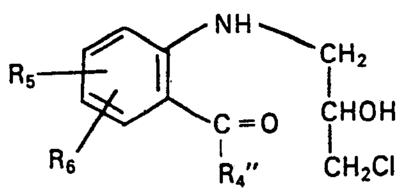

XIIIa

worin $R_4''$, $R_5$ und $R_6$ obige Bedeutung besitzen, umgesetzt, und in diesen gewünschtenfalls anschliessend auf an sich bekannte Weise die Aminogruppe alkyliert. Die Alkylierung kann beispielsweise nach den aus der Literatur bekannten Verfahren der reduktiven Carbonyl-Aminierung erfolgen, beispielsweise durch Umsetzung mit einem niederen Aldehyd in Gegenwart eines Reduktionsmittels, vorzugsweise Ameisensäure. Die erhaltenen Verbindungen der Formel XIII

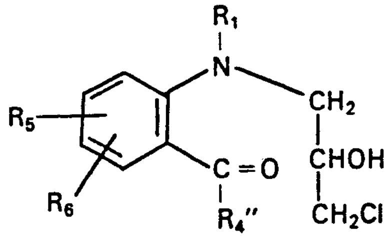

XIII

worin $R_1$, $R_4''$, $R_5$ und $R_6$ obige Bedeutung besitzen, werden anschliessend durch Behandeln mit Natronlauge in die entsprechenden Epoxidverbindungen umgesetzt, welche durch mehrstündiges Erhitzen mit Ammoniak in einem inerten Lösungsmittel, beispielsweise einem niederen Alkohol wie Methanol, zu Benzodiazocin-Verbindungen der Formel XIVa

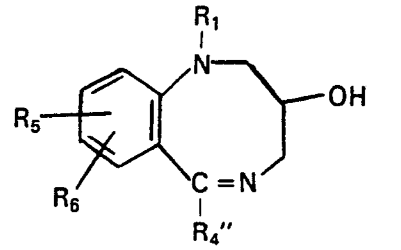

XIVa

worin $R_1$, $R_4''$, $R_5$ und $R_6$ obige Bedeutung besitzen, cyclisiert werden. Die Verbindungen der Formel XIVa können dann durch Behandlung mit $SOCl_2$, beispielsweise Kochen in $SOCl_2$ unter Rückfluss, in die Benzodiazepinverbindungen der Formel IX überführt werden. Dieses Verfahren eignet sich insbesondere zur Herstellung solcher Verbindungen, worin $R_4$ Thiophen darstellt.

Zur Herstellung von Verbindungen IX und X, bzw. deren Gemischen kann man auch ausgehen von Anthranilsäureestern oder -nitrilen der Formel XV

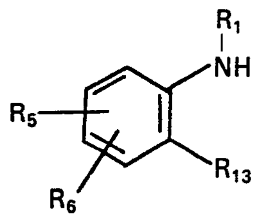

XV

worin $R_1$, $R_5$ und $R_6$ obige Bedeutung besitzen und $R_{13}$ für eine niedere Alkoxycarbonylgruppe oder CN steht. Diese werden zunächst mit Epichlorhydrin zu Verbindungen der Formel XVI

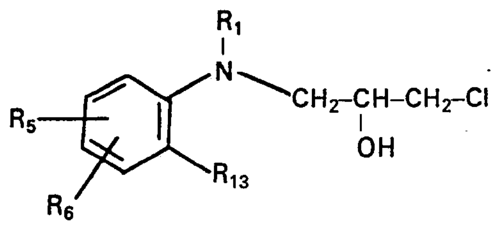

XVI

worin $R_1$, $R_5$, $R_6$ und $R_{13}$ obige Bedeutung besitzen, umgesetzt, welche anschliessend zu Lactonen oder Iminolactonen der Formel XVII

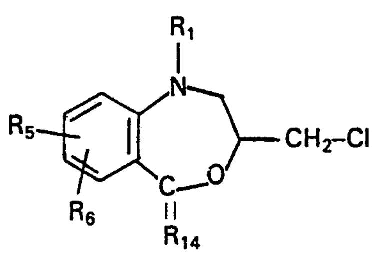

XVII

worin $R_1$, $R_5$ und $R_6$ obige Bedeutung besitzen und $R_{14}$ Sauerstoff oder Imino bedeutet, cyclisiert werden, beispielsweise durch Behandeln mit fein gemahlenem Kaliumhydroxid in Äther. Als Nebenprodukt bilden sich unter diesen Reaktionsbedingungen die den Verbindungen der Formel XVI entsprechenden Epoxide, welche jedoch durch Behandeln mit Salzsäure wieder in die Verbindungen der Formel XVI zurückverwandelt werden können. Aus Nitrilen erhaltene Iminolactone der Formel XVII ($R_{14}$ = NH) werden anschliessend hydrolytisch in die entsprechenden Lactone ($R_{14}$ = O) überführt. Die Lactone der Formel XVII werden mit durch Umsetzen von Verbindungen der Formel XVII

$R_4$-Br XVIII

worin $R_4$ obige Bedeutung besitzt, mit Lithiumbutyl in situ erhaltenen metallierten Verbindungen umgesetzt zu Verbindungen der Formel XIX

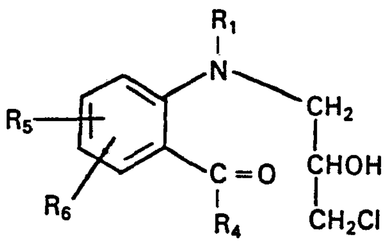

XIX

worin $R_1$, $R_4$, $R_5$ und $R_6$ obige Bedeutung besitzen. Diese werden durch Umsetzen mit einem Alkaliphthalimid und anschliessend Hydrolyse der Phthalimidgruppe zur Aminogruppe in Verbindungen der Formel XX

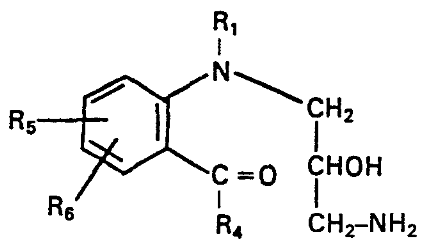

XX

worin $R_1$, $R_4$, $R_5$ und $R_6$ obige Bedeutung besitzen, überführt. Letztere werden durch mehrstündiges Erhitzen auf Temperaturen von 80–100 °C in einem inerten Lösungsmittel, beispielsweise einem aromatischen Kohlenwasserstoff wie Toluol, zu Benzodiazocin-Verbindungen der Formel XIV

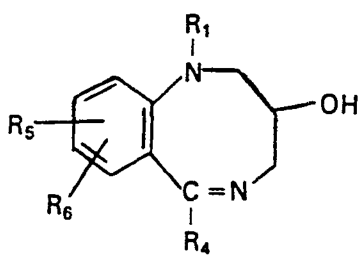

XIV

worin $R_1$, $R_4$, $R_5$ und $R_6$ obige Bedeutung besitzen, cyclisiert. Die Verbindungen der Formel XIV können dann durch Behandeln mit Triphenylphosphin / Tetrachlorkohlenstoff / Methylenchlorid in ein Gemisch der isomeren Verbindungen der Formel IX und X überführt werden.

Sofern die Substituenten in dem Benzodiazepingerüst nicht Alkyloxy- oder Alkylthiogruppen enthalten, können Ausgangsverbindungen der Formel II, worin $R_1$ Wasserstoff ist, auch dadurch erhalten werden, dass man Verbindungen der Formel II, worin $R_1$ Alkyl, vorzugsweise Methyl bedeutet, mit Jodwasserstoffsäure in an sich bekannter Weise entalkyliert. Die Reaktion kann in konzentrierter Jodwasserstoffsäure bei Temperaturen zwischen 50 und 100 °C durchgeführt werden.

Die erfindungsgemässen 2-Acylaminomethyl -5- heteroaryl -1H-2,3-dihydro-1,4-benzodiazepin-Verbindungen und ihre pharmakologisch akzeptablen Salze zeichnen sich gegenüber bekannten 1,4-Benzodiazepinderivaten durch ein pharmakologisch neuartiges Wirkungsprofil aus und zeigen insbesondere ausgeprägte analgetische Eigenschaften neben psychopharmakologischen, diuretischen und antiarrhytmischen Wirkungen bei geringer Toxizität.

Aufgrund ihrer ausgeprägten analgetischen Eigenschaften sind die erfindungsgemässen neuen Verbindungen nützliche Analgetica.

Die analgetischen Eigenschaften der Verbindungen zeigen sich in pharmakologischen Tests an kleinen Nagern und an Affen. So kann nachgewiesen werden, dass die Verbindungen der Formel I die Schmerzschwelle bei Säugetieren heraufzusetzen vermögen. Dies wird insbesondere nachgwiesen in zwei pharmakologischen Standard-Testmethoden, dem Brennstrahltest an der Maus und dem Arthritisschmerztest an der Ratte.

Beschreibung der pharmakologischen Untersuchungsmethoden.

1. Bestimmung der minimalen toxischen Dosis.

Bei männlichen Mäusen von 20–25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder toxische Symptome beobachtet werden, werden weiteren Mäusen zunehmend geringere Dosen verabreicht, bis keine toxischen Symptome mehr auftreten. Die niedrigste Dosis, welche toxische Symptome hervorruft, wird als minimale toxische Dosis angegeben.

2. Arthritisschmerztest an der Ratte.

Männlichen Ratten vom Stamm OFA mit einem Gewicht von 160–180 g werden durch Gaben von 20 mg/kg i.p. Pentobarbitalnatrium anästhesiert. 0,1 ml einer Suspension von Mycobakterium Smegmae (SI043) in Paraffinöl (0,6 mg Mycobakt./0,1 ml Öl) werden intracutan in die linke Hinterpfote injiziert. 14 Tage später, wenn sich besonders in der rechten Hinterpfote eine ausgeprägte sekundäre Arthritis entwickelt hat, werden die Wirkungen der Testsubstanzen untersucht. 30 Minuten vor Verabreichung der Testsubstanzen wird eine Kontrollmessung vorgenommen, indem das Fussgelenk der rechten Hinterpfote dreimal

gebeugt wird und die Anzahl der Lautgebungen gezählt wird. Ratten, welche nicht reagieren, werden ausgesondert. 3 Stunden nach oraler Gabe der Testsubstanzen wird die Beugeprozedur wiederholt. Tiere, welche entweder nur einmal oder überhaupt nicht Laut geben, werden als geschützt gegen Schmerzen angesehen. Zwischen 9 und 20 Ratten werden pro Dosis verwendet, und die $ED_{50}$ (95% Vertrauensbereich) wird nach der Methode von Litchfield und Wilcoxon (1949) bestimmt. Als $ED_{50}$ wird die Dosis bezeichnet, welche einen Schutz in 50% der behandelten Tiere erzeugt.

3. Brennstrahl-Test an der Maus.

Die Methode basiert auf der von D'Amour und Smith beschriebenen Arbeitsweise (1941). Es werden jedoch gefütterte männliche und weibliche Mäuse mit 16–25 g Körpergewicht anstelle von Ratten verwendet. 30 Minuten vor der Behandlung mit der Testsubstanz wird jede Maus einzeln in ein zylindrisches Gefäss gesetzt, so dass sie sich nicht umdrehen und vorwärts bewegen kann. Ihr Schwanz ragt in einer engen Rinne liegend aus dem Gefäss heraus. Ein bestimmter Punkt des Schwanzes jedes Tieres (ungefähr 35 mm von der Schwanzwurzel entfernt) wird der Bestrahlungshitze einer Lampe bekannter Stärke und Temperatur ausgesetzt, die sich direkt unter dem Schwanz befindet. Die Zeit in Sekunden, die die Maus braucht, um den Schwanz aus dem Lichtstrahl zu schnellen, wird zweimal bestimmt, einmal 30 und einmal 15 Minuten vor subkutaner Verabreichung der Testsubstanz (10 mg/kg). Die Mäuse, deren Reaktionszeiten um mehr als 25% abweichen, werden ausgeschieden. Die Reaktionszeiten werden erneut nach 15 und 30 Minuten nach der Behandlung gemessen und eine Ausdehnung der Reaktionszeiten auf mehr als 75% der durchschnittlichen Vorbehandlungswerte derselben Maus wird als analgetischer Effekt angesehen. Als $ED_{50}$ (95% Vertrauensbereich) jeder Testsubstanz 30 Minuten nach Verabreichung wird die Dosis angesehen, welche die Vorbehandlungsreaktionszeit um mehr als 75% in 50% der Tiere verlängert. Berechnung erfolgt nach der Methode von Litchfield und Wilcoxon (1949).

Die Verbindungen der Formel I zeigen in den vorstehend beschriebenen pharmakologischen Tests in einem Dosisbereich von 0,1–100 mg/kg analgetische Wirkungen.

Die folgende Tabelle gibt nach den vorstehend beschriebenen Testmethoden erhaltene Ergebnisse wieder.

Die für die Verbindungen der Formel I angegebenen Beispielsnummern beziehen sich auf die nachstehenden Herstellungsbeispiele.

| Test Substanz der Formel I Beispiel Nr. | Hemmung des Arthritisschmerzes an der Ratte $ED_{50}$ mg/kg p.o. | Tail flick Test an der Maus $ED_{50}$ mg/kg s.c. | Minimale toxische Dosis an der Maus mg/kg p.o. |
|---|---|---|---|
| 11 | ~ 2 | 2,8 | 300 |
| 7 | 8,5 | | >300 |
| 6 | 2 | | 300 |
| 1 | >32 <56 | 1 | 200 |
| 5 | ~10 | 2,7 | 300 |
| 12 | 32 | | >300 |
| 13 | >32 <56 | 10 | 200 |
| 14 | >32 <56 | 5 | 100 |
| 84 | 18 | | >300 |
| 87 | >18 <32 | 2,6 | |
| 88 | >18 <32 | 8,3 | |
| 89 | 10 | | |

Zur Verwendung als Arzneistoffe können sowohl die freien Basen als auch deren pharmakologisch annehmbare Säureadditionssalze eingesetzt werden. Als Säureadditionssalze eignen sich Salze von solchen Säuren, deren Anionen bei den in Frage kommenden Dosierungen nicht toxisch sind. Zur Salzbildung mit Verbindungen der allgemeinen Formel I eignen sich beispielsweise Chlorwasserstoffsäure, Bromwasserstoffsäure, Schwefelsäure, Phosphorsäure, Methansulfonsäure, Äthansulfonsäure, Benzolsulfonsäure, p-Toluolsulfonsäure, Zitronensäure, Essigsäure, Milchsäure, Bernsteinsäure, Maleinsäure, Fumarsäure, Apfelsäure, Weinsäure, Benzoesäure, Phenylessigsäure und Mandelsäure.

Die Verbindungen der Formel I können in pharmazeutischen Gebrauchsformen verabreicht werden, welche etwa 0,1–100 mg Aktivsubstanz pro Einzeldosis enthalten. Die verwendete Dosierung wird selbstverständlich der zu behandelnden Spezies und den individuellen Erfordernissen angepasst werden. Im allgemeinen werden jedoch bei Testtieren schmerzhemmende Wirkungen mit Dosen zwischen 0,1 und 100 mg/kg erhalten. Zur Behandlung von Schmerzen bei Menschen und grösseren Säugetieren sind z.B. Präparate mit 0,25 bis 50 mg, insbesondere 1 bis 50 mg, Aktivsubstanz pro Einzeldosis geeignet. Parenterale Formulierungen werden im allgemeinen weniger Aktivsubstanz enthalten als orale Präparate.

Die Verbindungen der Formel I können allein oder in Kombination mit pharmazeutisch anwendbaren Träger- und/oder Hilfsstoffen in Form fester oder flüssiger Dosierungsformen verwendet werden. Als Beispiele fester Präparate seien oralapplizierbare Präparate wie Tabletten, Kapseln, Pulver, Granulate oder Dragees genannt oder auch Suppositorien. Feste Präparate können pharmazeutisch übliche anorganische Trägerstoffe wie Talkum und/oder organische Trägerstoffe wie Milchzucker oder Stärke neben pharmazeutisch üblichen Hilfsmitteln, beispielsweise Gleitmitteln wie Magnesiumstearat enthalten. Flüssige Präparate wie Lösungen, Suspensionen oder Emulsionen können die üblichen Verdünnungsmittel wie Wasser,Öle oder Vaseline und/oder Suspensionsmittel wie Polyoxyethylenglykol und dergleichen enthalten. Es können auch zusätzlich weitere Hilfsstoffe zugegeben werden wie z.B. Konservierungsmittel, Stabilisierungsmittel und Netzmittel.

Die nachfolgenden Beispiele erläutern die Herstellung der neuen Verbindungen der allgemeinen Formel I sowie der bisher nicht bekannten neuen Zwischenprodukte. Sie sollen jedoch den Umfang der Erfindung in keiner Weise beschränken.

In der Tabelle werden bei Salzformen eventuell eingeschlossene Mengen an Wasser, Aceton, Äthanol oder dgl. angeführt.

Beispiel 1:

1-Methyl-2-[(furan-3-carbonyl)-aminomethyl] -5-(3-thienyl)-1H-2,3-dihydro- 1,4- benzodiazepin.

A) 42,6 g $N_1$- (Thiophen -3- carbonyl) -$N_2$-methyl- $N_2$-phenyl -2- hydroxy -1,3- diaminopropan wurden in 126 ml Phosphoroxidchlorid 5 Stunden unter Rückfluss gekocht. Anschliessend wurde die Reaktionslösung auf eine Mischung aus 250 g Eis/250 ml konzentrierter Salzsäure und 250 ml Methylenchlorid gegeben.

Die Methylenchloridphase wurde abgetrennt, mehrmals mit Wasser gewaschen, und anschliessend solange mit 30%iger Natronlauge gewaschen bis alle sauren Bestandteile entfernt waren. Danach wurde wiederum mit Wasser neutral gewaschen, über Natriumsulfat getrocknet, filtriert und das Methylenchlorid im Vakuum eingedampft. Als Rückstand wurden 31,5 g eines Gemisches der beiden isomeren Verbindungen 1-Methyl -2- chlormethyl -5- (3'-thienyl) -1H- 2,3-dihydro -1,4- benzodiazepin und 1-Methyl -3-chlor -6- (3'- thienyl) -1,2,3,4- tetrahydro -1,5-benzodiazocin erhalten.

B) Das vorstehende Gemisch wurde in 230 ml Methanol gelöst und mit 21,6 g Kaliumphthalimid und 6 g Kaliumjodid 12 Stunden unter Rückfluss gekocht. Darauf wurde das Methanol im Vakuum abdestilliert und der Rückstand in Methylenchlorid aufgenommen, filtriert und wiederum eingedampft. Der Rückstand wurde mit Toluol und Methylenchlorid über 300 g Aluminiumoxid der Aktivitätsstufe I filtriert. Es wurden 30,9 g 1- Methyl -2- phthalimidomethyl -5- (3'-thienyl) -1H- 2,3- dihydrobenzodiazepin erhalten.

C) Die vorstehende Phthalimidverbindung wurde mit 8,7 g Hydrazinhydrat in 300 ml Äthanol 4 Stunden unter Rückfluss erhitzt. Die Lösung wurde filtriert und das Lösungsmittel im Vakuum abdestilliert. Der Rückstand wurde in verdünnter Salzsäure (20%) aufgenommen und nochmals filtriert. Darauf wurde mit Methylenchlorid extrahiert und anschliessend wurde die saure, wässrige Lösung mit konzentrierter Natronlauge bis zur alkalischen Reaktion versetzt. Die ausgeschiedene Base wurde in Methylenchlorid gelöst, und die Lösung mit gesättigter Kochsalzlösung neutral gewaschen, über Natriumsulfat getrocknet und filtriert. Danach wurde das Lösungsmittel abdestilliert, der Rückstand (13,4 g) in Äther gelöst und mit einer Lösung von Chlorwasserstoff-Gas in Äther versetzt. Das kristallin ausgeschiedene 1- Methyl -2- amonimethyl -5- (3'-thienyl) -1H- 2,3- dihydro -1,4- benzodiazepin -dihydrochlorid wurde abgesaugt und mit Essigester und Äther gut gewaschen. Schmelzpunkt 188–207 °C (unter Zersetzung), Ausbeute 16,9 g.

D)10,9 g des vorstehenden Hydrochlorids wurden in 130 ml Methylenchlorid suspendiert und die Suspension mit 14,3 ml Triäthylamin versetzt. Dann wurden unter Eiskühlung 4,5 g Furan -3- carbonylchlorid in 50 ml Methylenchlorid langsam zugetropft. Die Reaktionslösung wurde nach dem Zutropfen 2 Stunden bei Raumtemperatur gerührt. Anschliessend wurde die Lösung mit Wasser, Ammoniaklösung (10%), wiederum Wasser und Kochsalzlösung gewaschen, über Natriumsulfat getrocknet und filtriert. Dann wurde das Lösungsmittel im Vakuum abdestilliert. Das zurückbleibende 1- Methyl -2- [(furan-3-carbonyl)-aminomethyl]-5-(3-thienyl)-1H- 2,3-dihydro - 1,4 - benzodiazepin wurde in Äther/Essigester 1:1 gelöst und mit einer Lösung von Chlorwasserstoff-Gas in Äther versetzt. Dann wurde das kristallin ausgeschiedene Hydrochlorid abgesaugt und aus Aceton umkristallisiert. Schmelzpunkt des Hydrochlorids 200–202,5 °C, Ausbeute: 7,9 g

Beispiel 2:

1-Methyl-2-[(furan-2-carbonyl)-aminomethyl]-5-(2'-furyl)-1H-2,3-dihydro-1,4-benzodiazepin.

A) 273 g $N_1$- Furan -2- carbonyl -$N_2$- methyl - $N_2$ - phenyl -2- hydroxy -1,3- diaminopropan wurden in 810 ml Phosphoroxidchlorid 1,5 Stunden unter Rückfluss gekocht. Nach beendeter Reaktion wurde die Hauptmenge an $POCl_3$ abdestilliert (ca. 750 ml) und der Rückstand auf 500 g Eis gegeben und 500 ml Methylenchlorid zugesetzt. Die organische Phase wurde abgetrennt und wiederholt mit Wasser gewaschen. Anschliessend wurde durch Zugabe von konzentrierter Natronlauge das Cyclisierungsprodukt als Base freigesetzt. Nach üblicher Aufarbeitung wurden 249 g Rohprodukt erhalten. Diese Rohbase wurde in 750 ml Äther gelöst, und die Lösung filtriert und

das Filtrat mit einer Lösung von Chlorwasserstoff-Gas in Äther versetzt. Das kristallin ausgeschiedene Hydrochlorid des Cyclisierungsprodukts wurde abgesaugt. Die Kristalle wurden in Wasser gelöst und durch Zusatz von konzentrierter Natronlauge wurde wiederum die Base erhalten. Diese wurde in Äther gelöst, die Lösung mit Wasser gewaschen, über Natriumsulfat getrocknet und filtriert. Nach Abdestillieren des Lösungsmittels wurden 179,9 g eines öligen Rückstandes erhalten welcher aus einem Gemisch aus 1-Methyl -2- chlormethyl-5- (2'-furyl) -1H-2,3-dihydro-1,4- benzodiazepin und 1-Methyl-3-chlor - 6- (2'-furyl)1,2,3,4-tetrahydro-1,5-benzodiazocin bestand.

B) Ohne Trennung oder weitere Reinigung wurden 179,9 g des vorstehenden Gemisches in 1200 ml Methanol mit 130 g Kaliumphthalimid und 36 g Kaliumjodid 12 Stunden unter Rückfluss gekocht. Dann wurde das Methanol abdestilliert und der Rückstand in 500 ml Methylenchlorid aufgenommen. Nach Zugabe von 50 g γ-Tonerde wurde die Mischung 4 Stunden gerührt. Dann wurde die Lösung vom Rückstand abgesaugt und eingedampft. Das als öliges Rohprodukt erhaltene 1-Methyl -2- phthalimido-5- (2'-furyl)-1H-2,3-dihydro-1,4- benzodiazepin (211,4 g) wurde ohne weitere Reinigung in die nächste Reaktionsstufe eingesetzt.

C) 38,5 g 1-Methyl -2- phthalimido-5- (2'-furyl) -1H-2,3-dihydro-1,4- benzodiazepin wurden mit 140 ml Salzsäure (24%) 7,5 Stunden unter Rückfluss gekocht. Die Reaktionslösung wurde filtriert und das Filtrat mit Methylenchlorid (100 ml) extrahiert. Die saure, wässrige Phase wurde dann mit konzentrierter Natronlauge bis zur alkalischen Reaktion versetzt und mit Methylenchlorid extrahiert. Die Methylenchloridphase wurde mit Wasser gewaschen, über Natriumsulfat getrocknet, filtriert und im Vakuum eingedampft. Die als Rückstand verbleibende Aminverbindung (18,5 g) wurde in Äther gelöst und mit einer Lösung von Chlorwasserstoff-Gas in Äther versetzt. 1-Methyl -2- aminomethyl-5- (2'-furyl) -1H-2,3-dihydro-1,4- benzodiazepin-dihydrochlorid fiel in Form von orange gefärbten Kristallen aus, welche abfiltriert, mit Aceton warm gerührt, abgesaugt und getrocknet wurden. Schmelzpunkt 210 °C, Ausbeute: 21,8 g.

D) 32,8 g 1-Methyl -2-aminomethyl-5- (2'-furyl) -1H-2,3-dihydro-1,4- benzodiazepin-dihydrochlorid wurden in 450 ml Methylenchlorid suspendiert und mit 46 ml Triäthylamin versetzt. Unter Eiskühlung wurden 14,4 g Furan-2-carbonsäurechlorid in 50 ml Methylenchlorid zugesetzt. Nach üblichem Aufarbeiten wurde das gebildete 1-Methyl-2-[(furan-2-carbonyl)-aminomethyl]-5-(2'-furyl)-1H-2,3-dihydro-1,4-benzodiazepin als Hydrochlorid isoliert. Schmelzpunkt 239–241 °C (unter Zersetzung), Ausbeute: 17 g.

Beispiel 3:

1-Methyl-2-[(thiophen-2-carbonyl) -aminomethyl]-5- (2'-thienyl)- 1H-2,3-dihydro-1,4-benzodiazepin.

A) 203 g $N_1$- (Thiophen-2-carbonyl) -$N_2$-methyl -$N_2$- phenyl-2-hydroxy -1,3- diaminopropan wurden in 410 ml Phosphoroxichlorid 20 Stunden unter Rückfluss gekocht. Die Reaktionsmischung wurde wie in Beispiel 1 oder 2 beschrieben aufgearbeitet. Das rohe Cyclisierungsprodukt wurde in Äther gelöst, die Lösung filtriert und mit einer Lösung von Chlorwasserstoff-Gas in Äther versetzt. Es schieden sich Kristalle von 1-Methyl -2- chlormethyl-5- (2'-thienyl) -1H-2,3-dihydro -1,4- benzodiazepin-Hydrochlorid ab, welche aus Aceton/Isopropanol umkristallisiert wurden. Schmelzpunkt 163–164,5 °C, Ausbeute: 98,3 g.

B) 26,4 g des vorstehenden Hydrochlorids wurden in 100 ml Dimethylformamid mit 10,4 g Natriumazid auf 100 °C erwärmt und 3 Stunden bei dieser Temperatur gehalten. Dann wurde das Dimethylformamid im Vakuum abdestilliert und der Rückstand in 100 ml Toluol aufgenommen und mit gesättigter Kochsalzlösung gewaschen. Nach dem Trocknen über Natriumsulfat und Filtrieren wurde das Lösungsmittel im Vakuum destilliert. Es wurden 20,1 g 1-Methyl -2- azidomethyl -5- (2-thienyl) -1H -2,3- dihydro-1,4-benzodiazepin erhalten.
IR-Spektrum: 2120 $cm^{-1}$.

C) 20,1 g der vorstehenden Azidomethylverbindung wurden in 100 ml Methanol gelöst und nach Zugabe von 4,4 ml Triäthylamin und 10 ml Hydrazinhydrat in 300 ml Methanol portionsweise mit ca. 10 g Raney-Nickel versetzt. Nach 4 Stunden wurde der Katalysator abgetrennt und die Reaktionslösung eingedampft. Der Rückstand wurde in Methylenchlorid gelöst und die Lösung mit gesättigter Kochsalzlösung gewaschen. Das nach üblichem Aufarbeiten erhaltene 1-Methyl -2- aminomethyl-5- (2'-thienyl) -1H-2,3-dihydro-1,4- benzodiazepin wurde in üblicher Weise in das Dihydrochlorid überführt. Schmelzpunkt 175 °C (unter Zersetzung), Ausbeute 16,6 g.

D) 16,6 g des vorstehenden Dihydrochlorids wurden mit 20,5 ml Triäthylamin in 400 ml Methylenchlorid gelöst und mit 7,3 ml Thiophen-2-carbonsäurechlorid in üblicher Weise analog wie in Beispiel 1D oder 2D beschrieben umgesetzt und nach üblichem Aufarbeiten wurde das gebildete 1-Methyl-2-[ (thiophen-2-carbonyl) -aminomethyl] -1H-2,3- dihydro-1,4-benzodiazepin als Hydrochlorid erhalten. Schmelzpunkt 173–197 °C, Ausbeute 6 g.

Beispiel 4:

1-Methyl -2- [ (furan -2- carbonyl) -aminomethyl] -5- (2'- thienyl) -1H- 2,3- dihydro -1,4-benzodiazepin.

A) 20,3 g 2- (2'-Aminobenzoyl) -thiophen wurden mit 10,2 g Epichlorhydrin und 6 g Essigsäure 6 Stunden auf 60–70 °C erwärmt. Diese Reaktionsmischung wurde anschliessend auf Wasser gegossen und mit Methylenchlorid extrahiert. Die organische Phase wurde abgetrennt, neutral gewaschen, getrocknet, filtriert und eingedampft.

Es wurden 25 g 2-{2'-[N- (3-Chlor-2-hydroxypropyl) -amino]-benzoyl} -thiophen als öliges Rohprodukt erhalten. Dieses wurde ohne weitere Reinigung mit 32 ml Ameisensäure und 16 ml 37%iger Formaldehydlösung 2,5 Stunden auf dem Wasserbad erhitzt. Anschliessend wurde die Lösung auf Eis gegossen und mit Chloroform extrahiert. Die organische Phase wurde mit gesättigter Natriumkarbonatlösung bis zur neutralen Reaktion gewaschen. Anschliessend wurde die Chloroformphase getrocknet, filtriert und eingedampft. Man erhielt 24 g 2-{2'-[N- (3-Chlor-2-hydroxypropyl) -methylamino]-benzoyl} -thiophen. Dieses wurde mit 3,5 g Natriumhydroxid und 6,8 ml Wasser in 50 ml Dioxan und 50 ml Isopropanol 10 Stunden bei Raumtemperatur umgesetzt. Nach Abziehen des organischen Lösungsmittels im Vakuum wurde das ölige Rohprodukt in Methylenchlorid gelöst und die Lösung mit Wasser neutral gewaschen, über Natriumsulfat getrocknet und filtriert. Das Methylenchlorid wurde abdestilliert und man erhielt 12 g 2-{2-[N-(2,3-Epoxypropyl)-methylamino]-benzoyl} -thiophen. Dieses wurde mit 36 g Ammoniak in 1200 ml Methanol durch 10stündiges Erhitzen auf 150 °C im Stahlautoklaven cyclisiert. Nach dem Abdestillieren des Lösungsmittels wurden 9 g 1-Methyl -3- hydroxy-6-thienyl -1,2,3,4- tetrahydro -1,5- benzodiazocin erhalten. Durch 2stündiges Kochen mit $SOCl_2$ unter Rückfluss wurden 10 g rohes 1-Methyl -2- chlormethyl-5-(2'-thienyl)-1H-2,3-dihydro-1,4-benzodiazepin-Hydrochlorid erhalten. Nach Umkristallisieren aus Aceton/Isopropanol wurden 8 g 1-Methyl -2- chlormethyl-5- (2'-thienyl)-1H-2,3-dihydro-1,4-benzodiazepin-hydrochlorid mit einem Schmelzpunkt von 163–164 °C erhalten.

B) Die vorstehende 2-Chlormethyl-Verbindung wurde wie in Beispiel 3 beschrieben in die entsprechende 2-Aminomethyl-Verbindung bzw. deren Dihydrochlorid (Schmelzpunkt 175 °C unter Zersetzung) überführt.

C) Das vorstehend erhaltene 1-Methyl -2- aminomethyl -5- (2'-thienyl) -1H-2,3-dihydro -1,4-benzodiazepin-dihydrochlorid wurde in üblicher Weise mit Furan-2-carbonsäurechlorid analog Beispiel 2D umgesetzt. Nach üblichem Aufarbeiten wurde das 1-Methyl-2-[ (furan-2-carbonyl) -aminomethyl] -5- (2-thienyl) -1H-2,3-dihydro -1,4- benzodiazepin-Hydrochlorid erhalten. Fp 221–225 °C.

Beispiel 5:

8-Methoxy-1-methyl-2-benzoylaminomethyl--5- (3'-thienyl) -1H-2,3-dihydro -1,4- benzodiazepin.

A) 16 g 8-Methoxy -1- methyl-2-azidomethyl-5-(3'-thienyl) -1H-2,3-dihydro -1,4- benzodiazepin (hergestellt aus $N_1$- (thiophen-3-carbonyl) -$N_2$-methyl-$N_2$- (3-methoxyphenyl) -2-hydroxy-1,3-diaminopropan analog Beispiel 3A–B) wurden in 200 ml Methanol gelöst und bei Raumtemperatur mit 12 ml 1,3-Dimercaptopropan und 16 ml Triäthylamin versetzt. Die Reaktionslösung wurde 48 Stunden bei Raumtemperatur gerührt. Dann wurde das Lösungsmittel abdestilliert und der Rückstand in 200 ml Äther gelöst. Die ätherische Lösung wurde filtriert und mit verdünnter Salzsäure (10%) extrahiert. Die saure, wässrige Lösung wurde mit Natronlauge (20%) bis zur alkalischen Reaktion versetzt. Dann wurde mit Äther extrahiert. Die ätherische Lösung wurde mit gesättigter Kochsalzlösung gewaschen, über Natriumsulfat getrocknet, filtriert und das Lösungsmittel abgezogen. Es wurden 9,9 g rohes 8-Methoxy-1-methyl-2-aminomethyl-5- (3'-thienyl) -1H-2,3-dihydro -1,4- benzodiazepin erhalten. Das Rohprodukt konnte ohne weitere Reinigung für die folgende Umsetzung eingesetzt werden. Es konnte in üblicher Weise durch Behandeln mit Chlorwasserstoff-Gas in Äther in das Dihydrochlorid überführt werden. Schmelzpunkt des Dihydrochlorids 190–211 °C (unter Zersetzung).

B) 9,5 g 8-Methoxy -1- methyl-2-aminomethyl-5- (3'-thienyl) -1H-2,3-dihydro -1,4- benzodiazepin wurden mit 3,2 g Triäthylamin in 120 ml Methylenchlorid gelöst. Darauf wurde zu der Lösung unter Eiskühlung tropfenweise eine Lösung von 4,9 g Benzoylchlorid in 20 ml Methylenchlorid gegeben. Die Reaktionslösung wurde noch 2 Stunden bei Raumtemperatur gerührt und dann mit Wasser, danach mit Ammoniaklösung (10%) und wieder mit Wasser gewaschen. Nach dem Trocknen über Natriumsulfat wurde filtiert und das Lösungsmittel im Vakuum abdestilliert. Es wurden 9 g rohes 8-Methoxy -1- methyl-2-benzoylaminomethyl-5- (3'-thienyl) -1H-2,3-dihydro -1,4- benzodiazepin als Rückstand erhalten. Das Rohprodukt wurde in einem Gemisch aus Äther und Essigsäureäthylester (1:1) gelöst. Durch Zugabe einer Lösung von Chlorwasserstoff-Gas in Äther wurde das Hydrochlorid ausgefällt. Es wurde abgesaugt und mit Aceton heiss ausgerührt. Ausbeute: 7,3 g des Hydrochlorids, Schmelzpunkt 233–234 °C.

Beispiel 6:

1-Methyl-2-[ (4'-trifluormethylbenzoyl) -aminomethyl]-5- (3'-thienyl) -1H-2,3-dihydro -1,4-benzodiazepin.

A) 10 g 1-Methyl -2- azidomethyl-5- (3'-thienyl) -1H-2,3-dihydro -1,4- benzodiazepin (hergestellt aus $N_1$- (Thiophen-3-carbonyl) -$N_2$-methyl-$N_2$-phenyl -2- hydroxy-1,3-diaminopropan analog Beispiel 3A–B) wurden in 200 ml konzentrierter Salzsäure gelöst und bei einer Innentemperatur von 0–3 °C mit 20 g Zinn-II-chlorid-dihydrat versetzt. Die Reaktionsmischung wurde 30 Minuten unter Eiskühlung und anschliessend 1 Std. bei Raumtemperatur gerührt. Dann wurden in Abständen von jeweils 2 Stunden jeweils weitere 10 g Zinn-II-chloriddihydrat zugesetzt. Nach 7 Stunden wurde die Reaktionslösung mit Methylenchlorid extrahiert und die Methylenchloridphase anschliessend mit Natronlauge (20%) gewaschen, über Natriumsulfat getrocknet und filtriert. Dann wurde das Lösungsmittel in Vakuum abdestilliert. Als Rückstand wurden 6,5 g rohes

1-Methyl -2- aminomethyl-5- (3'-thienyl) -1H-2,3-dihydro -1,4- benzodiazepin erhalten.

B)6 g 1-Methyl -2- aminomethyl-5- (3'-thienyl) -1H-2,3-dihydro -1,4- benzodiazepin wurden in 50 ml Toluol gelöst. Dann wurden 4,4 g 4-Trifluormethylbenzosäure zugesetzt und nach 30 Minuten 7 g Triäthylamin zugegeben. Zu dieser Reaktionslösung wurde unter Rühren eine Lösung von 1,6 g Phosphortrichlorid in 20 ml Toluol getropft, wobei ein Temperaturanstieg auf 40–50 °C eintrat. Die Reaktionslösung wurde noch 1 Stunde auf 80 °C erwärmt und anschliessend auf 500 ml Wasser gegossen. Durch Zusatz von konzentrierter Natronlauge wurde ein pH-Wert von 10 eingestellt. Die organische Phase wurde abgetrennt und die wässrige, alkalische Phase nochmals mit Methylenchlorid extrahiert. Die vereinigten, organischen Phasen wurden in üblicher Weise aufgearbeitet durch Waschen mit Wasser, Trocknen über Natriumsulfat, Filtrieren und Eindampfen. Aus dem aus dem Rückstand erhaltenen rohen 1-Methyl-2-[(4'-trifluormethylbenzoyl) -aminomethyl] -5- (3'-thienyl) -1H-2,3-dihydro -1,4- benzodiazepin wurde in üblicher Weise das Hydrochlorid hergestellt und aus Essigsäureäthylester kristallisiert. Ausbeute 7,5 g, Schmelzpunkt des Hydrochlorids 185–188 °C.

Beispiel 7:

1-Methyl-2-[ (4-cyanbenzoyl) -aminomethyl]-5- (3'-thienyl) -1H-2,3-dihydro -1,4- benzodiazepin.

15,4 g 4-Cyanbenzosäure wurden in 300 ml Methylenchlorid gelöst, die Lösung auf 0–5 °C abgekühlt und mit 14,6 ml Triäthylamin versetzt. Dann wurden innerhalb von 5–10 Minuten 10 ml Chlorameisensäureäthylester zugetropft und die Reaktionsmischung wurde noch 30 Minuten bei einer Temperatur von 0–5 °C gerührt. Anschliessend wurden 28,5 g 1-Methyl -2- aminomethyl-5- (3'-thienyl) -1H-2,3-dihydro -1,4- benzodiazepin (Herstellung siehe Beispiel 1A–C) gelöst in 200 ml Methylenchlorid so zugetropft, dass die Temperatur zwischen 0 und 5 °C beibehalten wurde. Anschliessend wurde die Reaktionslösung noch 4 Stunden bei Raumtemperatur gerührt. Die Reaktionsmischung wurde sodann in üblicher Weise aufgearbeitet. Es wurden 37,6 g der rohen Titelverbindung erhalten. Diese wurden in bekannter Weise in das Hydrochlorid überführt. Schmelzpunkt 234-239 °C, Ausbeute 31 g.

Beispiel 8:

8-Methoxy-1-methyl-2-[ (4'-cyanbenzoyl) -aminomethyl]-5- (3'-thienyl) -1H-2,3-dihydro -1,4- benzodiazepin.

12,7 g 2-Chlor -1- methylpyridiniumjodid wurden in 350 ml Methylenchlorid bei Raumtemperatur unter Rühren suspendiert und mit 14 ml Triäthylamin und 9,5 g 4-Cyanbenzosäure versetzt. Nach 15 Minuten wurde eine Lösung von 14,1 g 8-Methoxy -1- methyl-2-aminomethyl-5- (3'-thienyl) -1H-2,3-dihydro -1,4- benzodiazepin (Herstellung siehe Beispiel 5A) in 50 ml Methylenchlorid innerhalb von 15 Minuten zugetropft. Nach weiteren 30 Minuten wurden 300 ml Wasser zugegeben und die Mischung mit einer geringen Menge wässriger Ammoniaklösung (20%) bis zur schwach alkalischen Reaktion versetzt. Nach üblichem Aufarbeiten wurden 23,2 g rohe Titelverbindung als Rückstand erhalten. Dieser Rückstand wurde nach einander mit Äther, Methylenchlorid und Äthanol an 150 g Kieselgel chromatographiert. Die erhaltene Titelbase wurde in bekannter Weise in das Hydrochlorid überführt, dieses zeigte nach Ausrühren mit heissem Aceton einen Schmelzpunkt von 256–259 °C, Ausbeute 17,3 g.

Beispiel 9:

8-Methoxy -1- methyl-2-[ (furan-3-carbonyl) -aminomethyl]-5-(3'-thienyl) -1H-2,3-dihydro -1,4-benzodiazepin.

7,5 g 8-Methoxy -1- methyl-2-aminomethyl-5- (3'-thienyl) -1H-2,3-dihydro -1,4- benzodiazepin (Herstellung siehe Beispiel 5A) wurden in 50 ml Methylenchlorid gelöst und unter Stickstoff als Inertgas mit 10 ml einer 2,5-molaren Lösung von Trimethylaluminium in n-Hexan versetzt. Die Reaktionslösung wurde 15 Minuten bei Raumtemperatur gerührt. Dann wurde eine Lösung von 3,5 g Furan-3-carbonsäureäthylester in 20 ml Methylenchlorid zugetropft. Die Reaktionslösung wurde 50 Stunden auf 35–40 °C erwärmt. Anschliessend wurde das Rekationsgemisch durch vorsichtiges Zugeben einer Lösung von Ammoniumchlorid und Natriumchlorid in Wasser unter Eiskühlung zersetzt. Die entstandene organische Phase wurde abgetrennt und mehrmals mit gesättigter Kochsalzlösung gewaschen. Nach üblichem Aufarbeiten wurden 7,3 g 8-Methoxy -1- methyl-2-[ (furan-3-carbonyl) -aminomethyl]-5- (3'-thienyl) -1H-2,3-dihydro -1,4- benzodiazepin-hydrochlorid erhalten. Schmelzpunkt 237–238,5 °C.

Beispiel 10:

1-Methyl-2-[ (thiophen-3-carbonyl) -aminomethyl]-5-(3'-pyridyl) -1H-2,3-dihydro -1,4-benzodiazepin.

A) Zu einer auf 60 °C erwärmten Mischung aus 123,5 g N-Methylanthranilsäuremethylester und 42,6 g Eisessig werden 107 ml Epichlorhydrin unter Rühren zugetropft. Die Mischung wird weitere 16 Stunden bei 60 °C gerührt, anschliessend werden weitere 17 ml Epichlorhydrin zugetropft und noch weitere 4 Stunden bei 60 °C gerührt. Dann wird das Reaktionsgemisch auf Raumtemperatur abgekühlt, auf Eis/Wasser gegossen und mit Methylenchlorid extrahiert. Die Methylenchloridphase wird mit gesättigter Natriumbicarbonatlösung und Wasser gewaschen, über Natriumsulfat getrocknet, filtriert und eingedampft. Als Rückstand werden 218 g rohe N-Methyl-N (3-chlor-2-hydroxypropyl) -anthranilsäuremethylester erhalten.

B) 218 g des vorstehend erhaltenen Esters werden in 1,5 l trockenen Äther gegeben und unter Eiskühlung mit 51 g gemahlenem Kaliumhydroxid versetzt. Die Mischung wird 24 Stunden bei

Raumtemperatur gerührt. Anschliessend wird die Lösung abdekantiert, filtriert und eingedampft. Das im Rückstand verbleibende rohe 3-Chlormethyl -1- methyl-1,2-dihydro -3H-5H-1,4-benzazoxepin-5-on wird aus Äther kristallisiert und die Kristalle im Vakuum bei 50 °C getrocknet. Ausbeute 54,8 g.

C) 31,4 ml 3-Brom-pyridin werden in 300 ml absulutem Äther unter Feuchtigkeitsausschluss gelöst und die Lösung auf −50 °C abgekühlt. Unter Stickstoffatmosphäre werden langsam 200 ml einer 1,6molaren Lösung von n-Butyllithium in Hexan zugetropft und die Reaktionsmischung anschliessend 40 Minuten bei −50 °C gerührt. Die so hergestellte 3-Lithiumpyridyllösung wird bei ca. −60 °C zu einer Lösung von 54,2 g der unter B) hergestellten Verbindung in 400 ml absolutem Tetrahydrofuran getropft und das Reaktionsgemisch anschliessend 1 Stunde bei −60 °C gerührt. Dann wird bei dieser Temperatur langsam eine Lösung von 20 ml Wasser in 150 ml Tetrahydrofuran zugetropft. Das Reaktionsgemisch lässt man auf Raumtemperatur erwärmen und verdünnt dann mit ca. 200 ml Toluol. Die organische Phase wird abgetrennt, mit gesättigter Natriumchloridlösung gewaschen, über Natriumsulfat getrocknet, filtriert und eingedampft. Als Rückstand verbleiben 87 g Rohprodukt, welches durch Chromatographie an 850 g Kieselgel, Laufmittel Cyclohexan/Essigsäureäthylester 3:7, gereinigt wird. Es werden 39,0 g 2-{2-[N-(3-Chlor-2-hydroxypropyl) -methylamino]-benzoyl} -pyridin als Öl erhalten.

D) 39 g der vorstehend erhaltenen Verbindung werden in 314 ml Dimethylformamid gelöst und mit 32 g Kaliumphtalimid und 4,9 ml Pyridin versetzt. Die Reaktionsmischung wird 2 Stunden unter Feuchtigkeitsausschluss bei einer Temperatur von 120 °C gerührt. Dann wird das Dimethylformamid abgezogen und der Rückstand in Wasser und Methylenchlorid aufgenommen. Die organische Phase wird abgetrennt, mit Wasser gewaschen, über Natriumsulfat getrocknet, filtriert und eingedampft. Als Rückstand verbleiben 51,0 g 2-{2-[N- (3- Phthalimido -2- hydroxypropyl) -methylamino]-benzoyl} -pyridin.

E) 51,0 g der vorstehend erhaltenen Phthalimidverbindung werden in 612 ml konzentrierter Salzsäure 16 Stunden unter Rückfluss erhitzt, wobei von Zeit zu Zeit Chlorwasserstoff in das Reaktionsgefäss geleitet wird. Anschliessend wird die Salzsäure weitgehend abgezogen, der Rückstand in Wasser aufgenommen, unter Eiskühlung mit verdünnter Natronlauge alkalisch gestellt und mit Methylenchlorid extrahiert. Die organische Phase wird abgetrennt, mit gesättigter Natriumchloridlösung gewaschen, über Natriumsulfat getrocknet, filtriert und eingedampft. Es werden 29,8 g rohes 2-{2-[N- (3-Amino-2-hydroxypropyl) -methylamino]-benzoyl} -pyridin erhalten.

F) 29,8 g des vorstehend erhaltenen Amins werden in 259 ml Toluol 8 Stunden auf 90 °C unter Stickstoffatmosphäre erhitzt. Die Lösung wird auf Raumtemperatur abgekühlt, filtriert und eingedampft. Als Rückstand verbleiben 27,4 g 1-Methyl-3-hydroxy-6- (2-pyridyl) -1,2,3,4-tetrahydro -1,5- benzodiazocin.

G) 25,8 g der vorstehend erhaltenen 3-Hydroxybenzodiazocin-Verbindung werden in 161 ml Methylenchlorid gelöst und 190 ml Tetrachlorkohlenstoff und 26,6 g Triphenylphosphin zugegeben. Das Reaktionsgemisch wird 3 Stunden unter Rückfluss gerührt, wobei nach einer Stunde noch weitere 6,4 g und nach 1 1/2 Stunden noch ein weiteres Gramm Triphenylphosphin zugegeben werden. Anschliessend wird das Lösungsmittel abdestilliert und der Rückstand durch Chromatographie an Kieselgel, Laufmittel 1. Toluol, 2. Cyclohexan/Essigsäureäthylester 4:6, gereinigt. Es werden 40 g Rohprodukt erhalten, welches in 500 ml Äther aufgenommen wird. Hierbei fällt Triphenylphosphinoxid als weisser Niederschlag aus. Von diesem wird abgesaugt und die Lösung eingeengt. Es werden 23,3 g eines Gemisches aus 1-Methyl -2- chlormethyl-5-(2-pyridyl) -1H-2,3-dihydro -1,4-benzodiazepin und 1-Methyl -3- chlor -6- (2-pyridyl) -1,2,3,4-tetrahydro -1,5- benzodiazocin erhalten.

H) Ohne Trennung oder weitere Reinigung werden 25 g des vorstehenden Gemisches in 205 ml Methanol mit 18 g Kaliumphthalimid und 1,43 g Kaliumjodid 6 Stunden unter Rühren am Rückfluss gekocht, wobei nach 2 Stunden noch weitere 5 g und nach 5 Stunden noch weitere 2 g Kaliumphthalimid zugegeben werden. Anschliessend wird das Methanol abdestilliert, der Rückstand in Wasser/Methylenchlorid aufgenommen, die organische Phase abgetrennt, mit Wasser gewaschen, über Natriumsulfat getrocknet, filtriert und eingedampft. Es werden 27,5 g 1-Methyl -2-phthalimido- 5-(2-pyridyl)-1H- 2,3-dihydro -1,4- benzodiazepin erhalten.

I) 27,5 g der vorstehenden Phthalimidverbindung werden in 325 ml Äthanol mit 7,3 ml Hydrazinhydrat und 35 ml konzentrierter Salzsäure 2 Stunden unter Rückfluss erhitzt. Das Äthanol wird abgezogen, der Rückstand mit verdünnter Salzsäure versetzt und die Lösung wird von dem gebildeten Niederschlag abgesaugt und mit Methylenchlorid extrahiert. Dann wird die wässrige Phase unter Eiskühlung mit verdünnter Natronlauge alkalisch gestellt und mit Methylenchlorid extrahiert. Die Methylenchloridphase wird mit gesättigter Natriumchloridlösung gewaschen, über Natriumsulfat getrocknet, filtriert und eingedampft. Es werden 12,8 g 1-Methyl -2- aminomethyl- 5-(2-pyridyl)-1H- 2,3- dihydro-1,4-benzodiazepin erhalten.

J) 4,2 g des vorstehend erhaltenen Amins werden in 120 ml Methylenchlorid und 2,7 ml Triäthylamin gelöst. Unter Eiskühlung und Rühren wird zu dieser Lösung langsam eine Lösung von 2,5 g Thiophen-3-carbonsäurechlorid in 5 ml Methylenchlorid zugetropft. Das Reaktionsgemsich wird mit Wasser versetzt, die Methylenchloridphase abgetrennt, mit verdünnter Natronlauge und mit Wasser gewaschen, über Natriumsulfat getrocknet, filtriert und eingedampft. Es werden 6,4 g Rohprodukt erhalten, welche durch

Chromathographie an 80 g Aluminiumoxyd (Aktivitätsstufe II/III), Laufmittel Toluol/Methylenchlorid, gereinigt. Es werden 5,2 g 1-Methyl-2-[(thiophen-3-carbonyl)-aminomethyl]-5-(2-pyridyl)-1H-2,3-dihydro-1,4-benzodiazepin erhalten. 5,2 g dieser Base werden in Aceton gelöst und die Lösung mit einer Lösung von 1,8 g Fumarsäure in Äthanol versetzt. Die Lösung wird eingeengt und der Rückstand in Aceton aufgenommen. Das Difumarat der Titelverbindung scheidet sich langsam kristallin ab. Die Kristalle werden 2 Tage im Vakuum bei 60 °C getrocknet. Ausbeute 3,5 g, Schmelzpunkt 175–177 °C.

Nach den in Beispiel 1–10 beschriebenen Verfahren können auch die in der folgenden Tabelle aufgeführten 2-Acylaminomethyl-5-heteroaryl-1H-2,3-dihydro-1,4-benzodiazepin-Verbindungen durch Acylierung der entsprechenden 2-Aminomethyl-5-heteroacyl-1H-2,3-dihydro-1,4-benzodiazepin-Verbindungen erhalten werden.

| Bsp. Nr. | $R_1$ | $R_2$ | n | $R_3$ | $R_4$ | $R_5$ | $R_6$ | Salz | Fp °C |
|---|---|---|---|---|---|---|---|---|---|
| 11 | $CH_3$ | H | 0 | phen. | 3-thien. | H | H | HCl | 211–215 |
| 12 | $CH_3$ | H | 0 | 4-$CF_3$-phen. | 3-thien. | 8-$OCH_3$ | H | HCl | 243–246 |
| 13 | $CH_3$ | H | 0 | 3-thien. | 3-thien. | 8-$OCH_3$ | H | HCl | 234–236 |
| 14 | $CH_3$ | H | 0 | 2-thien. | 3-thien. | 7,8–$OCH_2O$ | | HCl | 267–273 |
| 15 | $CH_3$ | H | 0 | 2-fur. | 3-thien. | 7,8–$OCH_2O$ | | HCl | 256–265 |
| 16 | $CH_3$ | H | 0 | 3-fur. | 3-thien. | 7,8–$OCH_2O$ | | HCl | 274–277 |
| 17 | $CH_3$ | H | 0 | 3-thien. | 3-thien. | 7,8–$OCH_2O$ | | HCl | 276–283 |
| 18 | $CH_3$ | H | 0 | phen. | 3-thien. | 7,8–$OCH_2O$ | | HCl | 264–270 |
| 19 | $CH_3$ | H | 0 | 3-CN-phen. | 3-thien. | 7,8–$OCH_2O$ | | HCl | 246–258 |
| 20 | $CH_3$ | H | 0 | 4-$CH_3O$-phen. | 3-thien. | 7,8–$OCH_2O$ | | HCl | 270–276 |
| 21 | $CH_3$ | H | 0 | 4-CN-phen. | 3-thien. | 7,8–$OCH_2O$ | | HCl · 0,2 $C_2H_5OH$ | 271–279 |
| 22 | $CH_3$ | H | 0 | 4-$NO_2$-phen. | 3-thien. | 7,8–$OCH_2O$ | | HCl | 266–271 |
| 23 | $CH_3$ | H | 2 | phen. | 3-thien. | 7,8–$OCH_2O$ | | HCl | 193–198 |
| 24 | $CH_3$ | H | 0 | 2-pyrid. | 3-thien. | 7,8–$OCH_2O$ | | 1,8 HCl · 1 $H_2O$ | 204–212 (Z) |
| 25 | $CH_3$ | H | 1 | 3-pyrid. | 3-thien. | 7,8-$OCH_2O$ | | HCl · 0,1 $(CH_3)_2CO$ · 0,7 $H_2O$ | 154–166 (Z) |
| 26 | $CH_3$ | H | 0 | 2-pyrrol. | 3-thien. | 7,8-$OCH_2O$ | | Base | 216–222 |
| 27 | $CH_3$ | H | 0 | 4-CN-phen. | 2-thien. | 7-$CH_3O$ | H | HCl | 252–257 |
| 28 | $CH_3$ | H | 1 | N-$CH_3$-4-$CH_3$-2-pyrrol. | 3-thien. | 7,8-$OCH_2O$ | | Base, am | 82– 88 |
| 29 | $CH_3$ | H | 1 | 4-$CF_3$-phen. | 3-thien. | H | H | HCl | 202–205 |
| 30 | $CH_3$ | H | 1 | 2-$CF_3$-phen. | 3-thien. | H | H | HCl | 225–228 |
| 31 | $CH_3$ | H | 0 | 5-$CH_3$-2-thien. | 3-thien. | H | H | HCl | 216–222 |
| 32 | $CH_3$ | H | 0 | 2-$CH_3O$-4-Cl-phen. | 3-thien. | H | H | HCl | 184–187 |
| 33 | $CH_3$ | H | 1 | 4-Cl-phen. | 3-thien. | H | H | HCl | 200–203 |
| 34 | $CH_3$ | H | 0 | 2-Cl-3-pyrid. | 3-thien. | H | H | 2HCl | 190–195 |
| 35 | $CH_3$ | H | 0 | N-$CH_3$-5-$CH_3$-2-pyrrol. | 3-thien. | H | H | Base | 90 |
| 36 | $CH_3$ | H | 2 | 4-$CH_3O$-phen. | 3-thien. | H | H | Base | Öl |
| 37 | $CH_3$ | H | 0 | 3,4-di-Cl-phen. | 3-thien. | H | H | HCl | 209–211 |
| 38 | $CH_3$ | H | 0 | phen. | 3-thien. | 7-$CH_3O$ | H | HCl | 197–203 |
| 39 | $CH_3$ | H | 0 | 4-CN-phen. | 3-thien. | 7-$CH_3O$ | H | HCl | 208–212 |
| 40 | $CH_3$ | H | 0 | 3-thien. | 3-thien. | 7-$CH_3O$ | H | Base | 157–162 |
| 41 | $CH_3$ | H | 0 | phen. | 3-thien. | 7-F | H | Base | 142–145 |
| 42 | $CH_3$ | H | 0 | 4-CN-phen. | 3-thien. | 7-F | H | Base | 173 |
| 43 | $CH_3$ | H | 0 | 3-fur. | 3-thien. | 7-F | H | HCl | 220–225 |
| 44 | $CH_3$ | H | 0 | 2-Cl-phen. | 3-thien. | 7-F | H | HCl | 223–227 |
| 45 | $CH_3$ | H | 0 | phen. | 3-thien. | 7-Cl | H | HCl | 193–196 |
| 46 | $CH_3$ | H | 1 | phen. | 3-thien. | 7-Cl | H | Base | Öl |
| 47 | $CH_3$ | H | 0 | 2-Cl-phen. | 3-thien. | 7-Cl | H | HCl | 214–217 |
| 48 | $CH_3$ | H | 0 | 3-Cl-phen. | 3-thien. | 7-Cl | H | HCl · 0,25 $(CH_3)_2CHOH$ | 204–207 |

| Bsp. Nr. | $R_1$ | $R_2$ | n | $R_3$ | $R_4$ | $R_5$ | $R_6$ | Salz | Fp °C |
|---|---|---|---|---|---|---|---|---|---|
| 49 | $CH_3$ | H | 1 | 3-Cl-phen. | 3-thien. | 7-Cl | H | HCl · 0,25 $H_2O$ am | 132–137 |
| 50 | $CH_3$ | H | 1 | 2-Cl-phen. | 3-thien. | 7-Cl | H | HCl · 0,1 $(CH_3)_2CHOH$ | 187–191 |
| 51 | $CH_3$ | H | 0 | 4-$NO_2$-phen. | 3-thien. | 7-Cl | H | Base 0,1 HCl | 197 |
| 52 | $CH_3$ | H | 0 | 4-CN-phen. | 3-thien. | 7-Cl | H | 1,2 HCl | 235–240 |
| 53 | $CH_3$ | H | 0 | 4-F-phen. | 3-thien. | 7-Cl | H | HCl | 235–238 |
| 54 | $CH_3$ | H | 0 | 3-fur. | 3-thien. | 7-Cl | H | HCl | 227–230 |
| 55 | $CH_3$ | H | 0 | 3-thien. | 3-thien. | 7-Cl | H | HCl | 205–209 |
| 56 | $CH_3$ | H | 1 | 3-thien. | 3-thien. | 7-Cl | H | HCl | 184–187 |
| 57 | $CH_3$ | H | 0 | 2-pyrid. | 3-thien. | 7-Cl | H | Base | Öl |
| 58 | $CH_3$ | H | 0 | phen. | 3-thien. | 8-$CH_3$ | H | HCl | 207–211 |
| 59 | $CH_3$ | H | 1 | phen. | 3-thien. | 8-$CH_3$ | H | HCl | 184–190 |
| 60 | $CH_3$ | H | 0 | 4-CN-phen. | 3-thien. | 8-$CH_3$ | H | HCl · 0,5 $H_2O$ | 152–156 |
| 61 | $CH_3$ | H | 2 | phen. | 3-thien. | 8-$CH_3$ | H | HCl · 0,3 $(CH_3)_2CHOH$ am | 129–134 |
| 62 | $CH_3$ | H | 0 | 4-F-phen. | 3-thien. | 8-$CH_3$ | H | HCl | 218–222 |
| 63 | $CH_3$ | H | 0 | 2-Cl-phen. | 3-thien. | 8-$CH_3$ | H | HCl · 0,25 $H_2O$ | 208–211 |
| 64 | $CH_3$ | H | 1 | 2-Cl-phen. | 3-thien. | 8-$CH_3$ | H | HCl am | 108–112 |
| 65 | $CH_3$ | H | 0 | 3-thien. | 3-thien. | 8-$CH_3$ | H | 1,05 HCl · 0,5 $H_2O$ | 216–220 |
| 66 | $CH_3$ | H | 0 | 3-fur. | 3-thien. | 8-$CH_3$ | H | HCl | 212–216 |
| 67 | $CH_3$ | H | 0 | 4-$NO_2$-phen. | 3-thien. | 8-$CH_3$ | H | HCl | 159–162 |
| 68 | $CH_3$ | H | 0 | 2-pyrid. | 3-thien. | 8-$CH_3$ | H | HCl · 0,4 $H_2O$ am | 136–138 |
| 69 | $CH_3$ | H | 1 | 3-thien. | 3-thien. | 8-$CH_3$ | H | Base | Öl |
| 70 | $CH_3$ | H | 0 | 4-$CH_3O$-phen. | 3-thien. | 8-$CH_3$ | H | HCl | 233–236 |
| 71 | H | H | 0 | 4-CN-phen. | 3-thien. | 8-F | H | 1,2 HCl · 0,25 $H_2O$, am | 131–135 |
| 72 | $CH_3$ | H | 0 | 4-CN-phen. | 3-thien. | 8-F | H | HCl | 212–216 |
| 73 | $CH_3$ | H | 0 | phen. | 3-thien. | 8-F | H | HCl | 199–203 |
| 74 | $CH_3$ | H | 0 | 4-$CF_3$-phen. | 3-thien. | 8-F | H | HCl | 205–209 |
| 75 | $CH_3$ | H | 0 | 3-fur. | 3-thien. | 8-F | H | Base | Öl |
| 76 | H | H | 0 | phen. | 3-thien. | 8-F | H | Base | Öl |
| 77 | $C_2H_5$ | H | 0 | 4-CN-phen. | 3-thien. | 8-F | H | HCl | 210–205 |
| 78 | n-$C_3H_7$ | H | 0 | 3-fur. | 3-thien. | 8-F | H | Base | Öl |
| 79 | n-$C_4H_9$ | H | 0 | 4-CN-phen. | 3-thien. | 8-F | H | HCl · 0,2 $(CH_3)_2CHOH$ | 179–183 |
| 80 | $CH_3$ | H | 0 | 4-F-phen. | 3-thien. | 8-F | H | HCl | 197–201 |
| 81 | $CH_3$ | H | 0 | 3-thien. | 3-thien. | 8-$OC_2H_5$ | H | HCl | 211–214 |
| 82 | $CH_3$ | H | 0 | 4-$CH_3O$-phen. | 3-thien. | 8-$OC_2H_5$ | H | HCl | 200-204 |
| 83 | $CH_3$ | H | 0 | phen. | 3-thien. | 8-$OC_2H_5$ | H | Base | Öl |
| 84 | $CH_3$ | H | 0 | 4-CN-phen. | 5-Cl-2-thien. | H | H | HCl | 221–232 |

| Bsp. Nr. | $R_1$ | $R_2$ | n | $R_3$ | $R_4$ | $R_5$ | $R_6$ | Salz | Fp °C |
|---|---|---|---|---|---|---|---|---|---|
| 85 | $CH_3$ | H | 0 | 3-fur. | 5-Cl-2-thien. | H | H | HCl | 215–227 |
| 86 | $CH_3$ | H | 0 | 4-CN-phen. | 4-Br-2-thien. | H | H | HCl | 255–258 |
| 87 | $CH_3$ | H | 0 | 3-fur. | 3-$CH_3$-2-thien. | H | H | HCl | 234–238 |
| 88 | $CH_3$ | H | 0 | phen. | 3-$CH_3$-2-thien. | H | H | HCl | 257–259 (Z) |
| 89 | $CH_3$ | H | 0 | 4-CN-phen. | 3-$CH_3$-2-thien. | H | H | HCl | 157–160 |
| 90 | $CH_3$ | H | 0 | phen. | 5-$CH_3$-2-thien. | 8-$CH_3$ | H | HCl | 227–230 |
| 91 | $CH_3$ | H | 0 | 4-CN-phen. | 5-$CH_3$-2-thien. | 8-$CH_3$ | H | HCl | 207–211 |
| 92 | $CH_3$ | H | 0 | 4-$NO_2$-phen. | 5-$CH_3$-2-thien. | 8-$CH_3$ | H | HCl | 191–197 |
| 93 | $CH_3$ | H | 1 | 2-Cl-phen. | 5-$CH_3$-2-thien. | 8-$CH_3$ | H | HCl | 204–206 |
| 94 | $CH_3$ | H | 1 | 3-thien. | 5-$CH_3$-2-thien. | 8-$CH_3$ | H | HCl | 233–235 |
| 95 | $CH_3$ | H | 0 | 3-fur. | 5-$CH_3$-2-thien. | 8-$CH_3$ | H | Base | 183–185 |
| 96 | $CH_3$ | H | 0 | 4-$CH_3O$-phen. | 3-Cl-2-thien. | H | H | HCl · 0,5 $H_2O$ | 209–216 |
| 97 | $CH_3$ | H | 0 | 3-fur. | 3-Cl-2-thien. | 8-$CH_3$ | H | HCl | 236–246 |
| 98 | $CH_3$ | H | 0 | 4-CN-phen. | 3-Cl-2-thien. | 8-$CH_3$ | H | HCl | 216–219 |
| 99 | $CH_3$ | H | 2 | phen. | 5-$CH_3$-2-thien. | 8-$CH_3$ | H | HCl · 0,35 $H_2O$ | 125 (S) |
| 100 | $CH_3$ | H | 0 | N-$CH_3$-2-pyrrol. | 5-$CH_3$-2-thien. | 8-$CH_3$ | H | HCl · 0,5 $H_2O$ | 175 |
| 101 | $CH_3$ | H | 0 | 2-thien. | 2-fur. | H | H | HCl | 173–176 |
| 102 | $CH_3$ | H | 0 | phen. | 2-fur. | 8-$CH_3O$ | H | HCl | 218–227 |
| 103 | $CH_3$ | H | 0 | phen. | 2-fur. | H | H | HCl | 201–203 |
| 104 | $CH_3$ | H | 0 | 3-fur. | 2-fur. | H | H | HCl · 0,5 $H_2O$ | 150 (S) |
| 105 | $CH_3$ | H | 0 | 3-CN-phen. | 2-fur. | H | H | HCl · 0,6 $H_2O$ | 165 (S) |
| 106 | $CH_3$ | H | 0 | 4-CN-phen. | 2-fur. | H | H | 1,7 HCl · 0,7 $H_2O$ | 180 (S) |
| 107 | $CH_3$ | H | 0 | 3-fur. | 3-fur. | 8-$CH_3O$ | H | HCl | 236–239 |
| 108 | $CH_3$ | H | 0 | 3-fur. | 3-fur. | 8-$CH_3$ | H | HCl | 244–252 |
| 109 | $CH_3$ | H | 0 | phen. | 3-fur. | 8-$CH_3$ | H | HCl | 228–235 |
| 110 | $CH_3$ | H | 0 | 3-thien. | 3-fur. | 8-$CH_3$ | H | HCl | 236–238 |
| 111 | $CH_3$ | H | 0 | 4-CN-phen. | 3-fur. | 8-$CH_3$ | H | HCl · 0,3 $H_2O$ | 259–261 |
| 112 | $CH_3$ | H | 0 | 3-CN-phen. | 3-fur. | 8-$CH_3$ | H | HCl | 193–195 |
| 113 | $CH_3$ | H | 0 | 3-fur. | 3-fur. | 8-$CH_3O$ | H | HCl | 236–239 |
| 114 | $CH_3$ | H | 0 | phen. | 3-fur. | 8-$CH_3O$ | H | HCl | 240–242 |
| 115 | $CH_3$ | H | 0 | 4-$CF_3$-phen. | 3-fur. | 8-$CH_3O$ | H | HCl | 240–244 |
| 116 | $CH_3$ | H | 0 | 4-$CH_3$-phen. | 3-fur. | H | H | HCl | 238–240 |
| 117 | $CH_3$ | H | 0 | 4-$CH_3O$-phen. | 3-fur. | H | H | Base | Öl |
| 118 | $CH_3$ | H | 1 | 2-Cl-phen. | 3-fur. | 8-$CH_3O$ | H | HCl | 152–156 |
| 119 | $CH_3$ | H | 0 | 3-fur. | 3-fur. | H | H | HCl | 222–225 |
| 120 | $CH_3$ | H | 0 | 4-CN-phen. | 3-fur | H | H | HCl | 173–178 (S) |
| 121 | $CH_3$ | H | 0 | phen. | 3-fur. | H | H | HCl | 235–237 |
| 122 | $CH_3$ | H | 0 | 3-fur. | 2-pyrid. | H | H | Base | 143–145 |
| 123 | $CH_3$ | H | 1 | 2-Cl-phen. | 2-pyrid. | H | H | p-Tos · 0,3 $H_2O$ | 132–135 |

| Bsp. Nr. | $R_1$ | $R_2$ | n | $R_3$ | $R_4$ | $R_5$ | $R_6$ | Salz | Fp °C |
|---|---|---|---|---|---|---|---|---|---|
| 124 | $CH_3$ | H | 0 | 4-CN-phen. | 2-pyrid. | H | H | p-Tos · 0,25 $H_2O$ | 201–204 |
| 125 | $CH_3$ | H | 0 | 4-CN-phen. | 3-pyrid. | H | H | p-Tos | 199–201 |
| 126 | $CH_3$ | H | 0 | 3-fur. | 3-pyrid. | H | H | di-Fum | 154–157 |
| 127 | $CH_3$ | H | 1 | 2-Cl-phen. | 4-pyrid. | 8-$CH_3O$ | H | 2,25 Mal | 176–178 |
| 128 | $CH_3$ | H | 0 | 4-CN-phen. | 4-pyrid. | 8-$CH_3O$ | H | Base | Öl |
| 129 | $CH_3$ | H | 0 | 3-fur. | 4-pyrid. | 8-$CH_3O$ | H | Base | Öl |
| 130 | $CH_3$ | H | 0 | phen. | 2-pyrid. | 7-$CH_3$ | H | Base | Öl |
| 131 | $CH_3$ | H | 0 | 3-fur. | 2-pyrid. | 7-$CH_3$ | H | Base | Öl |
| 132 | $CH_3$ | H | 0 | phen. | N-$CH_3$-2-pyrrol. | H | H | p-Tos | 180–182 |
| 133 | $CH_3$ | H | 1 | 2-Cl-phen. | N-$CH_3$-2-pyrrol. | H | H | Base | 135–136 |
| 134 | $CH_3$ | H | 0 | phen. | N-$CH_3$-3-pyrrol. | H | H | Base am | 93– 98 |
| 135 | $CH_3$ | H | 0 | phen. | 3-pyrrol. | H | H | Base | Öl |
| 136 | $CH_3$ | H | 0 | phen. | 2-thien. | 7-$CH_3O$ | H | 1,1 HCl · 0,4 $H_2O$ | 202–205 |
| 137 | $CH_3$ | H | 0 | 3-fur. | 2-thien. | 7-$CH_3O$ | H | 1,3 HCl · 0,5 $H_2O$ | 143–158 |
| 138 | $CH_3$ | H | 0 | 4-$CH_3O$-phen. | 3-thien. | 8-F | H | Base | Öl |
| 139 | $CH_3$ | H | 0 | phen. | 3-thien. | 7-$CH_3$ | 8-F | HCl · 0,2 $H_2O$ · 0,1 $(CH_3)_2CHOH$ | 183–187 |
| 140 | $CH_3$ | H | 0 | 3,4-$OCH_2O$-phen. | 3-thien. | 7-$CH_3$ | 8-F | HCl | 197–201 |
| 141 | $CH_3$ | H | 0 | 3,4-$OCH_2O$-phen. | 3-thien. | 7-F | 8-$CH_3$ | HCl | 212–218 |
| 142 | $CH_3$ | H | 0 | 4-$NH_2$-phen. | 3-thien. | 7-Cl | H | 1,85 HCl · 0,5 $H_2O$ am | 112–116 |
| 143 | $CH_3$ | H | 0 | 4-$CH_3CONH$-phen. | 3-thien. | 7-Cl | H | 1,05 HCl · 0,25 $H_2O$ | 178–182 |
| 144 | $CH_3$ | H | 0 | 4-$(CH_3)_2N$-phen. | 3-thien. | 7-Cl | H | 1,9 HCl · 0,45 $H_2O$ am | 122–128 |
| 145 | $CH_3$ | H | 1 | 4-F-phen. | 3-thien. | H | H | HCl | 223–229 |
| 146 | $CH_3$ | H | 1 | 2-F-phen. | 3-thien. | H | H | HCl | 198–200 |
| 147 | $CH_3$ | H | 1 | 2-F-phen. | 3-thien. | 7,8-$OCH_2O$ | | HCl | 227–234 |
| 148 | $CH_3$ | H | 0 | phen. | N-$CH_3$-3-pyrrol. | 8-$OCH_3$ | H | Base | Öl |

phen. = phenyl
fur. = furyl
thien. = thienyl
pyrrol. = pyrrolyl
pyrid. = pyridyl
HCl = Hydrochlorid
Base = freie Base
p-Tos. = p-Toluolsulfonat
Fum = Fumarat
Öl = ölig
(Z) = Zersetzung
(S) = Sintern
am = amorph
Mal = Maleinat

Beispiel I: Tabletten

Man stellt Tabletten mit folgender Zusammensetzung pro Tablette her.

| | |
|---|---|
| 1-Methyl-2-[ (furan-2-carbonyl) -aminomethyl]-5-(2'-furyl) -1H-2,3-dihydro-1,4-benzodiazepin-hydrochlorid | 25 mg |
| Maisstärke | 60 mg |
| Milchzucker | 130 mg |
| Gelatine (10% Lösung) | 6 mg |

Der Wirkstoff, Maisstärke und der Milchzucker werden mit einer 10%igen Gelatine-Lösung eingedickt. Die Paste wird zerkleinert, das erhaltene Granulat wird auf ein geeignetes Blech gebracht und bei 45 °C getrocknet. Das getrocknete Granulat wird durch eine Zerkleinerungsmaschine geleitet. Dann werden in einem Mixer die folgenden Substanzen zugemischt:

| | |
|---|---|
| Talkum | 5 mg |
| Magnesiumstearat | 5 mg |
| Maisstärke | 9 mg |

und die erhaltene Mischung sodann zu Tabletten von 240 mg verpresst.

Beispiel II: Suppositorien

Man stellt Suppositorien mit folgender Zusammensetzung her:

| | |
|---|---|
| 1-Methyl-2-[ (thiophen-3-carbonyl) -aminomethyl]-5-(3-thienyl) -1H-2,3-dihydro-1,4-benzodiazepin | 25 mg |
| Kakaobutter | 1975 mg |

Der Wirkstoff und die fein geriebene Suppositoriengrundmasse werden gründlich gemischt und dann geschmolzen. Aus der durch Rühren homogen gehaltenen Schmelze werden Suppositorien von 2 Gramm gegossen.

Beispiel III: Injektionslösung

Eine parenterale Gebrauchsform wird mit den folgenden Bestandteilen hergestellt:

| | | |
|---|---|---|
| 1-Methyl-2-[ (thiopen-2-carbonyl)-aminomethyl]-5-(3-thienyl) -1H-2,3-dihydro-1,4-benzodiazepin | | 10 % |
| Dimethylacetamid | | 10 % |
| Propylenglykol | | 50 % |
| Benzylalkohol | | 1,5% |
| Äthanol | | 10 % |
| Wasser für Injektionszwecke | ad | 100 % |

Der Wirkstoff wird in Dimethylacetamid gelöst und mit Benzylalkohol, Propylenglykol, Äthanol und Wasser versetzt. Man filtriert durch einen Kerzenfilter, füllt in geeignete Ampullen, verschliesst und sterilisiert.

**Patentansprüche für die Vertragsstaaten BE, CH, DE, FR, GB, IT, LI, LU, NL, SE**

1. 2-Acylaminomethyl -1H-2,3- dihydro -1,4-benzodiazepin-Verbindungen der allgemeinen Formel I

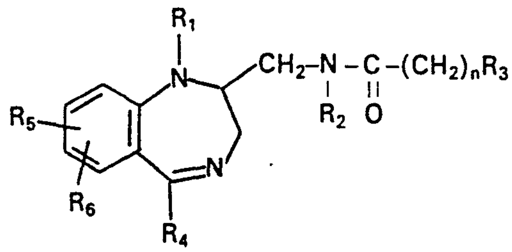

I

worin

$R_1$ Wasserstoff, Alkyl mit 1–4 Kohlenstoffatomen, Allyl, 2-Butenyl, 3-Butenyl oder Cyclopropylmethyl bedeutet,

$R_2$ Wasserstoff, Alkyl mit 1–4 Kohlenstoffatomen, Allyl, 2-Butenyl oder 3-Butenyl bedeutet,

n für 0, 1 oder 2 steht,

$R_3$ für eine der folgenden Gruppen a, b, c oder d steht,

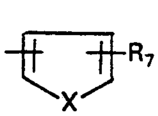

a

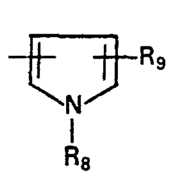

b

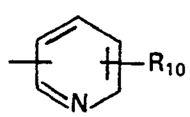

c

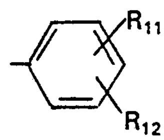

d

worin

X Sauerstoff oder Schwefel bedeutet,

$R_7$ Wasserstoff, Alkyl mit 1–4 Kohlenstoffatomen, Alkoxy mit 1–4 Kohlenstoffatomen, Nitro oder Halogen bedeutet,

$R_8$ Wasserstoff oder Alkyl mit 1–4 Kohlenstoffatomen bedeutet,

$R_9$ Wasserstoff oder Alkyl mit 1–4 Kohlenstoffatomen bedeutet,

$R_{10}$ Wasserstoff, Alkyl mit 1–4 Kohlenstoffatomen oder Chlor bedeutet,

$R_{11}$ Wasserstoff, Alkyl mit 1–4 Kohlenstoffatomen, Alkoxy mit 1–4 Kohlenstoffatomen, Hydroxy, Halogen, Alkylthio mit 1–4 Kohlenstoffatomen, Nitro, Trifluormethyl, Cyano, Amino, durch Alkyl mit 1–4 Kohlenstoffatomen mono- oder disubstituiertes Amino, Monoalkanoylamino mit 1–4 Kohlenstoffatomen, N-$C_1$ –$C_4$-Alkyl-N-$C_1$ – $C_4$ -alkanoylamino oder Alkanoyloxy mit 1–4 Kohlenstoffatomen bedeutet,

$R_{12}$ Wasserstoff, Alkyl mit 1–4 Kohlenstoffatomen, Alkoxy mit 1–4 Kohlenstoffatomen oder Halogen bedeutet, oder

$R_{11}$ und $R_{12}$ an benachbarte Kohlenstoffatome gebunden sind und zusamman Methylendioxy oder Äthylendioxy bedeuten,

$R_4$ für eine der vorgehend definierten Gruppen a, b oder c steht,

$R_5$ Wasserstoff, Alkyl mit 1–4 Kohlenstoffatomen, Alkoxy mit 1–4 Kohlenstoffatomen, Hydroxy, Halogen, Alkylthio mit 1–4 Kohlenstoffatomen, Nitro Trifluormethyl, Cyano, Amino, durch Alkyl mit 1–4 Kohlenstoffatomen mono- oder disubstituiertes Amino, Monoalkanoylamino mit

1–4 Kohlenstoffatomen, $N–C_1–C_4$-Alkyl- $N–C_1–C_4$ -Alkanoylamino oder Alkanoyloxy mit 1–4 Kohlenstoffatomen bedeutet, und

$R_6$ Wasserstoff, Alkyl mit 1–4 Kohlenstoffatomen, Alkoxy mit 1–4 Kohlenstoffatomen oder Halogen bedeutet, oder

$R_5$ und $R_6$ an benachbarte Kohlenstoffatome gebunden sind und zusammen Methylendioxy oder Äthylendioxy bedeuten, sowie deren optische Isomere und deren Säureadditonssalze.

2. 2-Acylaminomethyl -1H-2,3- dihydro -1,4-benzodiazepin-Verbindungen gemäss Anspruch 1, worin $R_1$, $R_2$, $R_4$, $R_5$ und $R_6$ obige Bedeutung besitzen und $R_3$ für die Gruppe d steht.

3. 2-Acylaminomethyl -1H-2,3- dihydro -1,4-benzodiazepin-Verbindungen gemäss Anspruch 1, worin $R_1$, $R_2$, $R_3$, $R_5$ und $R_6$ obige Bedeutung besitzen und $R_4$ für die Gruppe a steht.

4. 2-Acylaminomethyl -1H-2,3- dihydro -1,4-benzodiazepin-Verbindungen gemäss Anspruch 1–3, worin $R_3$, $R_4$, $R_5$ und $R_6$ obige Bedeutung besitzen und $R_1$ Wasserstoff, Alkyl mit 1–4 Kohlenstoffatomen oder Cyclopropylmethyl bedeutet, $R_2$ Wasserstoff oder Alkyl mit 1–4 Kohlenstoffatomen bedeutet und n für 0 oder 1 steht.

5. 2-Acylaminomethyl -1H-2,3- dihydro -1,4-benzodiazepin-Verbindungen gemäss Anspruch 1, worin

$R_1$ Wasserstoff oder Alkyl mit 1–4 Kohlenstoffatomen bedeutet,

$R_2$ Wasserstoff bedeutet,

n die in Anspruch 1 angegebene Bedeutung besitzt,

$R_3$ für eine Gruppe a steht, worin X die in Anspruch 1 angegebene Bedeutung besitzt und $R_7$ Wasserstoff, Alkyl mit 1–4 Kohlenstoffatomen oder, falls X Schwefel ist, auch Fluor, Chlor oder Brom bedeutet, oder für eine Gruppe b steht, worin $R_8$ und $R_9$ die in Anspruch 1 angegebene Bedeutungen besitzen, oder für eine Gruppe c steht, worin $R_{10}$ Wasserstoff bedeutet,

oder für eine Gruppe d steht, worin $R_{11}$ Wasserstoff, Alkyl mit 1–4 Kohlenstoffatomen, Alkoxy mit 1–4 Kohlenstoffatomen, Halogen oder Cyano und $R_{12}$ Wasserstoff, Alkyl mit 1–4 Kohlenstoffatomen, Alkoxy mit 1–4 Kohlenstoffatomen oder Halogen bedeuten oder $R_{12}$ und $R_{11}$ an benachbarte Kohlenstoffatome gebunden sind und gemeinsam Methylendioxy bedeuten,

$R_4$ für eine der vorstehend definierten Gruppen a, b oder c steht,

$R_5$ Wasserstoff, Alkyl mit 1–4 Kohlenstoffatomen, Alkoxy mit 1–4 Kohlenstoffatomen, Halogen oder Nitro bedeutet,

$R_6$ Wasserstoff, Alkyl mit 1–4 Kohlenstoffatomen, Alkoxy mit 1–4 Kohlenstoffatomen oder Halogen bedeutet oder $R_5$ und $R_6$ an benachbarte Kohlenstoffatome gebunden sind und gemeinsam Methylendioxy bedeuten.

6. 2-Acylaminomethyl -1H-2,3- dihydro -1,4-benzodiazepin-Verbindungen gemäss Anspruch 5, worin $R_1$, $R_2$, $R_3$, $R_4$ $R_5$ und $R_6$ obige Bedeutung besitzen und n für 0 oder, falls $R_3$ eine Gruppe d bedeutet, auch für 1 steht.

7. 2-Acylaminomethyl -1H-2,3- dihydro -1,4-benzodiazepin-Verbindungen gemäss Anspruch 5 oder 6, worin $R_1$, $R_2$, $R_3$, $R_5$, $R_6$ und n obige Bedeutung besitzen und $R_4$ für die Gruppe a steht.

8. 1-Methyl-2-[ (4-cyanobenzoyl) -aminomethyl]-5- 3'-thienyl) -1H-2,3-dihydro -1,4-benzodiazepin und dessen Säureadditionssalze gemäss Anspruch 1.

9. 2-Aminomethyl -1H-2,3- dihydro -1,4- benzodiazepin-Verbindungen der allgemeinen Formel IIa

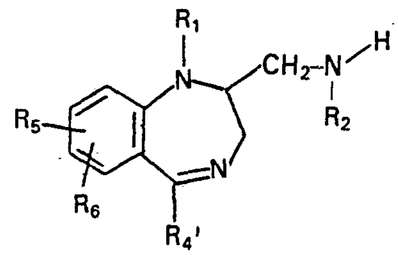

IIa

worin $R_1$, $R_2$, $R_5$ und $R_6$ die in Anspruch 1 angegebene Bedeutung besitzen,

$R_4'$ für eine der folgenden Gruppen a', b oder c steht

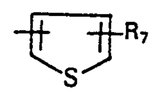
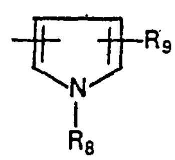
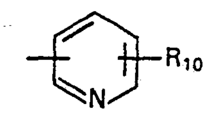

a' b c

worin $R_7$, $R_8$, $R_9$ und $R_{10}$ die in Anspruch 1 angegebene Bedeutung besitzen, sowie deren optische Isomere und deren Säureadditionssalze.

10. 2-Azidomethyl -1H-2,3- dihydro -1,4-benzodiazepin-Verbindungen der allgemeinen Formel IV

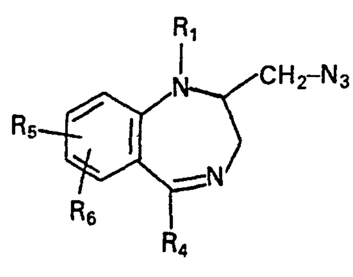

IV

worin $R_1$, $R_5$ und $R_6$ die in Anspruch 1 angegebene Bedeutung besitzen,

$R_4$ für eine der folgenden Gruppe a, b oder c steht

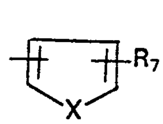
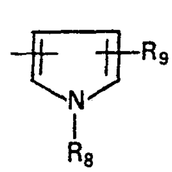
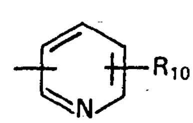

a b c

worin X $R_7$ $R_8$, $R_9$ und $R_{10}$ die in Anspruch 1 angegebene Bedeutung besitzen, sowie deren optische Isomere und deren Säureadditionssalze.

11. Verfahren zur Herstellung von 2-Acylaminomethyl -1H-2,3- dihydro -1,4- benzodiazepin-Verbindungen der allgemeinen Formel I

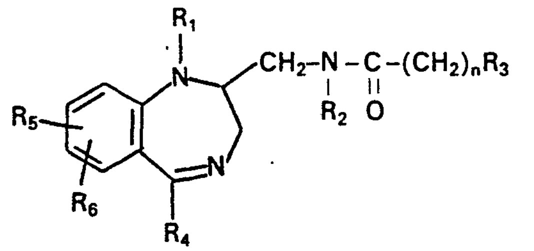

I

worin n, $R_1$, $R_2$, $R_3$, $R_4$, $R_5$ und $R_6$ die in Anspruch 1 angegebene Bedeutung besitzen sowie deren optischen Isomeren und deren Säureadditionssalzen, dadurch gekennzeichnet, dass man 2-Aminomethyl -1H-2,3- dihydro -1,4- benzodiazepin-Verbindungen der Formel II

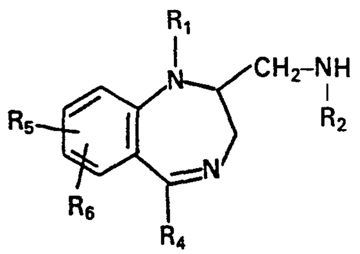

II

worin $R_1$, $R_2$, $R_4$, $R_5$ und $R_6$ obige Bedeutung besitzen, mit Säuren oder reaktionsfähigen Säurederivaten der Formel III

$R_3-(CH_2)_n-CO-Y$ III

worin $R_3$ und n obige Bedeutung besitzen und Y Hydroxy oder eine aminolytisch abspaltbare Gruppe bedeutet, acyliert und gegebenenfalls Verbindungen der Formel I, worin $R_2$ Wasserstoff bedeutet, zu Verbindungen der Formel I, worin $R_2$ Alkyl mit 1–4 Kohlenstoffatomen, Allyl, 2-Butenyl oder 3-Butenyl bedeutet, alkyliert und gegebenenfalls racemische Gemische von Verbindungen der Formel I in ihre optischen Isomeren auftrennt und gegebenenfalls freie Verbindungen der Formel I in ihre Säureadditionssalze überführt oder die Säureadditionssalze in die freien Verbindungen der Formel I überführt.

12. Arzneimittel enthaltend eine pharmakologisch wirksame Menge einer 2-Acylaminomethyl -1H-2,3- dihydro -1,4- benzodiazepin-Verbindung gemäss Anspruch 1 und übliche Hilfs-und/oder Trägerstoffe.

**Patentansprüche für den Vertragsstaat: AT**

1. Verfahren zur Herstellung von 2-Acylaminomethyl -1H-2,3- dihydro -1,4-benzodiazepin-Verbindungen der allgemeinen Formel I

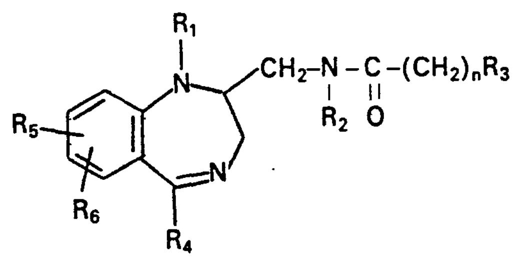

I

worin

$R_1$ Wasserstoff, Alkyl mit 1–4 Kohlenstoffatomen, Allyl, 2-Butenyl, 3-Butenyl oder Cyclopropylmethyl bedeutet,

$R_2$ Wasserstoff, Alkyl mit 1–4 Kohlenstoffatomen, Allyl, 2-Butenyl oder 3-Butenyl bedeutet,

n für 0, 1 oder 2 steht,

$R_3$ für eine der folgenden Gruppen a, b, c oder d steht

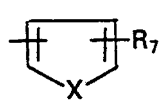

a

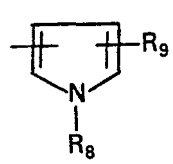

b

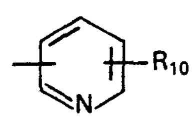

c

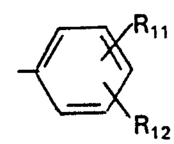

d

worin

X Sauerstoff oder Schwefel bedeutet,

$R_7$ Wasserstoff, Alkyl mit 1–4 Kohlenstoffatomen, Alkoxy mit 1–4 Kohlenstoffatomen, Nitro oder Halogen bedeutet,

$R_8$ Wasserstoff oder Alkyl mit 1–4 Kohlenstoffatomen bedeutet,

$R_9$ Wasserstoff oder Alkyl mit 1–4 Kohlenstoffatomen bedeutet,

$R_{10}$ Wasserstoff, Alkyl mit 1–4 Kohlenstoffatomen oder Chlor bedeutet,

$R_{11}$ Wasserstoff, Alkyl mit 1–4 Kohlenstoffatomen, Alkoxy mit 1–4 Kohlenstoffatomen, Hydroxy, Halogen, Alkylthio mit 1–4 Kohlenstoffatomen, Nitro, Trifluormethyl, Cyano, Amino, durch Alkyl mit 1–4 Kohlenstoffatomen mono, oder disubstituiertes Amino, Monoalkanoylamino mit 1–4 Kohlenstoffatomen, N-$C_1$ -$C_4$ -Alkyl -N-$C_1$ -$C_4$ -alkanoylamino oder Alkanoyloxy mit 1–4 Kohlenstoffatomen bedeutet,

$R_{12}$ Wasserstoff, Alkyl mit 1–4 Kohlenstoffatomen, Alkoxy mit 1–4 Kohlenstoffatomen oder Halogen bedeutet, oder

$R_{11}$ und $R_{12}$ an benachbarte Kohlenstoffatome gebunden sind und zusammen Methylendioxy oder Äthylendioxy bedeuten,

$R_4$ für eine der vorgehend definierten Gruppen a, b oder c steht,

$R_5$ Wasserstoff, Alkyl mit 1–4 Kohlenstoffatomen, Alkoxy mit 1–4 Kohlenstoffatomen, Hydroxy, Halogen, Alkylthio mit 1–4 Kohlenstoffatomen, Nitro, Trifluormethyl, Cyano, Amino, durch Alkyl mit 1–4 Kohlenstoffatomen mono- oder disubstituiertes Amino, Monoalkanoylamino mit 1–4 Kohlenstoffatomen, N–$C_1$ –$C_4$ -Alkyl –N–$C_1$ –$C_4$ -Alkanoylamino oder Alkanoyloxy mit 1–4 Kohlenstoffatomen bedeutet, und

$R_6$ Wasserstoff, Alkyl mit 1–4 Kohlenstoffatomen, Alkoxy mit 1–4 Kohlenstoffatomen oder Halogen bedeutet, oder

$R_5$ und $R_6$ an benachbarte Kohlenstoffatome gebunden sind und zusammen Methylendioxy oder Äthylendioxy bedeuten,

sowie deren optischen Isomeren und deren Säureadditionssalzen, dadurch gekennzeichnet, dass man 2-Aminomethyl -1H-2,3- dihydro -1,4-benzodiazepin-Verbindungen der Formel II

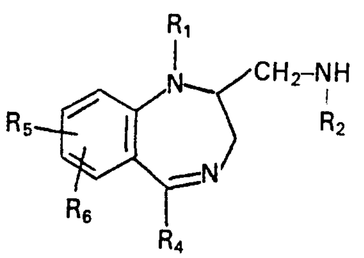

II

worin $R_1$, $R_2$, $R_4$, $R_5$ und $R_6$ obige Bedeutung besitzen, mit Säuren oder reaktionsfähigen Säurederivaten der Formel III

$$R_3–(CH_2)_n–CO–Y \qquad III$$

worin $R_3$ und n obige Bedeutung besitzen und Y Hydroxy oder eine aminolytisch abspaltbare Gruppe bedeutet, acyliert und gegebenenfalls Verbindungen der Formel I, worin $R_2$ Wasserstoff bedeutet, zu Verbindungen der Formel I, worin $R_2$ Alkyl mit 1–4 Kohlenstoffatomen, Allyl, 2-Butenyl oder 3-Butenyl bedeutet, alkyliert und gegebenenfalls racemische Gemische von Verbindungen der Formel I in ihre optischen Isomeren auftrennt und gegebenenfalls freie Verbindungen der Formel I in ihre Säureadditionssalze überführt oder die Säureadditionssalze in die freien Verbindungen der Formel I überführt.

2. Verfahren gemäss Anspruch 1, dadurch gekennzeichnet, dass 2-Acylaminomethyl -1H-2,3-dihydro -1,4- benzodiazepin-Verbindungen der Formel I hergestellt werden, worin $R_1$, $R_2$, $R_4$, $R_5$ und $R_6$ obige Bedeutung besitzen und $R_3$ für die Gruppe d steht.

3. Verfahren gemäss Anspruch 1, dadurch gekennzeichnet, dass 2-Axylaminomethyl -1H-2,3-dihydro -1,4-benzodiazepin-Verbindungen der Formel I hergestellt werden, worin $R_1$, $R_2$, $R_3$, $R_5$ und $R_6$ obige Bedeutung besitzen und $R_4$ für die Gruppe a steht.

4. Verfahren gemäss Anspruch 1–3, dadurch gekennzeichnet, dass 2-Acylaminomethyl -1H-2,3- dihydro -1,4- benzodiazepin-Verbindungen der Formel I hergestellt werden, worin $R_3$, $R_4$, $R_5$ und $R_6$ obige Bedeutung besitzen und $R_1$ Wasserstoff, Alkyl mit 1–4 Kohlenstoffatomen oder Cyclopropylmethyl bedeutet, $R_2$ Wasserstoff oder Alkyl mit 1–4 Kohlenstoffatomen bedeutet und n für 0 oder 1 steht.

5. Verfahren gemäss Anspruch 1, dadurch gekennzeichnet, dass 2-Acylaminomethyl -1H-2,3-dihydro -1,4- benzodiazepin-Verbindungen der Formel I hergestellt werden, worin

$R_1$ Wasserstoff oder Alkyl mit 1–4 Kohlenstoffatomen bedeutet,

$R_2$ Wasserstoff bedeutet,

n die in Anspruch 1 angegebene Bedeutung besitzt,

$R_3$ für eine Gruppe a steht, worin X die in Anspruch 1 angegebene Bedeutung besitzt und $R_7$ Wasserstoff, Alkyl mit 1–4 Kohlenstoffatomen oder, falls X Schwefel ist, auch Fluor, Chlor oder Brom bedeutet, oder für eine Gruppe b steht, worin $R_8$ und $R_9$ die in Anspruch 1 angegebene Bedeutungen besitzen, oder für eine Gruppe c steht, worin $R_{10}$ Wasserstoff bedeutet, oder für eine Gruppe d steht, worin $R_{11}$ Wasserstoff, Alkyl mit 1–4 Kohlenstoffatomen, Alkoxy mit 1–4 Kohlenstoffatomen, Halogen oder Cyano und $R_{12}$ Wasserstoff, Alkyl mit 1–4 Kohlenstoffatomen, Alkoxy mit 1–4 Kohlenstoffatomen oder Halogen bedeuten oder $R_{12}$ und $R_{11}$ an benachbarte Kohlenstoffatome gebunden sind und gemeinsam Methylendioxy bedeuten,

$R_4$ für eine der vorstehend definierten Gruppen a, b oder c steht,

$R_5$ Wasserstoff, Alkyl mit 1–4 Kohlenstoffatomen, Alkoxy mit 1–4 Kohlenstoffatomen, Halogen oder Nitro bedeutet,

$R_6$ Wasserstoff, Alkyl mit 1–4 Kohlenstoffatomen, Alkoxy mit 1–4 Kohlenstoffatomen oder Halogen bedeutet oder $R_5$ und $R_6$ an benachbarte Kohlenstoffatome gebunden sind und gemeinsam Methylendioxy bedeuten.

6. Verfahren gemäss Anspruch 5, dadurch gekennzeichnet, dass 2-Acylaminomethyl -1H-2,3-dihydro -1,4- benzodiazepin-Verbindungen der Formel I hergestellt werden, worin $R_1$, $R_2$, $R_3$, $R_4$, $R_5$ und $R_6$ obige Bedeutung besitzen und n für 0 oder, falls $R_3$ eine Gruppe d bedeutet, auch für 1 steht.

7. Verfahren gemäss Anspruch 5 oder 6, dadurch gekennzeichnet, dass 2-Acylaminomethyl -1H-2,3- dihydro -1,4- benzodiazepin-Verbindungen der Formel I hergestellt werden, worin $R_1$, $R_2$, $R_3$, $R_5$, $R_6$ und n obige Bedeutung besitzen und $R_4$ für die Gruppe a steht.

8. Verfahren gemäss Anspruch 1, dadurch gekennzeichnet, dass 1-Methyl-2-[ (4-cyanobenzoyl) -aminomethyl]-5- (3'-thienyl) -1H-2,3- dihydro -1,4- benzodiazepin und dessen Säureadditionssalze hergestellt werden.

9. Verfahren zur Herstellung von 2-Aminomethyl -1H-2,3- dihydro -1,4- benzodiazepin-Verbindungen der allgemeinen Formel IIa

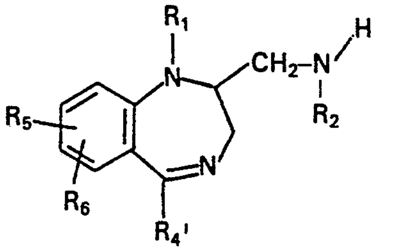

IIa

worin $R_1$, $R_2$, $R_5$ und $R_6$ die in Anspruch 1 angegebene Bedeutung besitzen,

$R_4'$ für eine der folgenden Gruppen a', b oder c steht

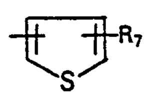 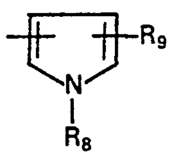 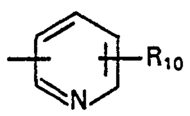

a' b c

worin $R_7$, $R_8$, $R_9$ und $R_{10}$ die in Anspruch 1 angegebene Bedeutung besitzen, sowie deren optischen Isomeren und deren Säureadditionssalzen, dadurch gekennzeichnet, dass man in Verbindungen der Formel IV

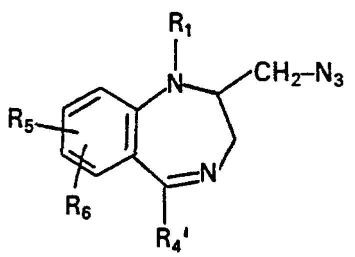

IV

worin $R_1$, $R_4'$, $R_5$ und $R_6$ obige Bedeutung besitzen, die Azidogruppe zur Aminogruppe reduziert oder in Verbindungen der Formel V

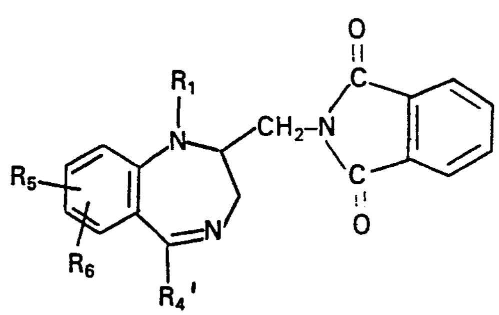

V

worin $R_1$, $R_4'$, $R_5$ und $R_6$ obige Bedeutung besitzen, die Phthalimidgruppe zur Aminogruppe hydrolisiert und gegebenenfalls die erhaltenen 2-Aminomethyl-Verbindungen alkyliert.

10. Verfahren zur Herstellung von 2-Azidomethyl -1H-2,3- dihydro -1,4- benzodiazepin-Verbindungen der allgemeinen Formel IV

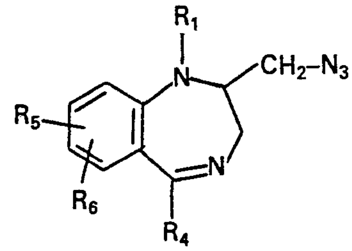

IV

worin $R_1$, $R_5$ und $R_6$ die in Anspruch 1 angegebene Bedeutung besitzen,

$R_4$ für eine der folgenden Gruppe a, b oder c steht

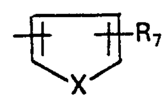 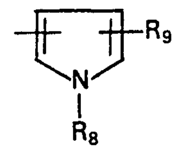 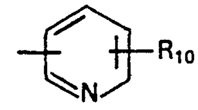

a b c

worin X, $R_7$, $R_8$, $R_9$ und $R_{10}$ die in Anspruch 1 angegebene Bedeutung besitzen, sowie deren optischen Isomeren und deren Säureadditionssalzen, dadurch gekennzeichnet, dass man Verbindungen der Formel IX und/oder Verbindungen der Formel X

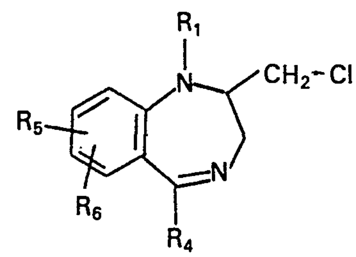

IX

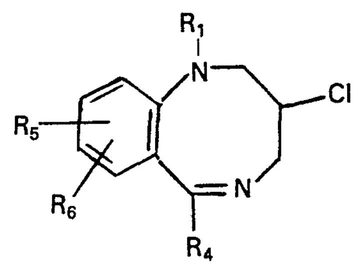

X

worin $R_1$, $R_4$, $R_5$ und $R_6$ obige Bedeutung besitzen, mit einem Alkalimetallazid umsetzt.

**Revendications pour les Etats Contractants: BE, CH, DE, FR, GB, IT, LI, LU, NL, SE**

1. 2-acylaminométhyl -1H-2,3- dihydro -1,4- benzodiazépines répondant à la formule générale I:

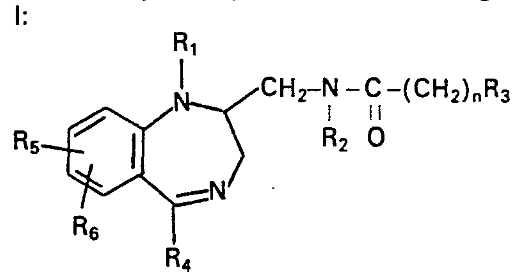

I

dans laquelle:

$R_1$ représente l'hydrogène, un alkyle avec 1 à 4 atomes de carbone, un allyle, un buténył-2, un buténylе-3 ou un cyclopropylméthyle,

$R_2$ représente l'hydrogène, un alkyle avec 1 à 4 atomes de carbone, un allyle, un buténylе-2 ou un buténylе-3;

n est 0, 1 ou 2,

$R_3$ est un des groupes a, b, c ou d suivants:

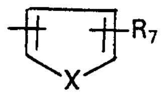
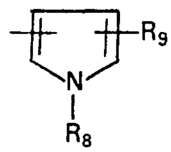
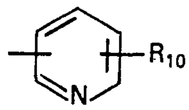
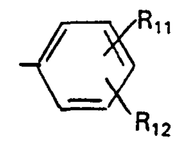

a b c d

où

X représente de l'oxygène ou du soufre,

$R_7$ représente l'hydrogène, un alkyle avec 1 à 4 atomes de carbone, un alcoxy avec 1 à 4 atomes de carbone, un nitro ou un halogène,

$R_8$ représente l'hydrogène ou un alkyle avec 1 à 4 atomes de carbone,

$R_9$ représente l'hydrogène ou un alkyle avec 1 à 4 atomes de carbone,

$R_{10}$ représente l'hydrogène, un alkyle avec 1 à 4 atomes de carbone ou du chlore,

$R_{11}$ représente l'hydrogène, un alkyle avec 1 à 4 atomes de carbone, un alcoxy avec 1 à 4 atomes de carbone, un hydroxy, un halogène, un alkylthio avec 1 à 4 atomes de carbone, un nitro, un trifluorométhyle, un cyano, un amino, un amino mono- ou disubstitué par un alkyle avec 1 à 4 atomes de carbone, un monoalcanoylamino avec 1 à 4 atomes de carbone, un N–$C_1$ à $C_4$-alkyl–N–$C_1$ à $C_4$-alcanoylamino ou un alcanoyloxy avec 1 à 4 atomes de carbone,

$R_{12}$ représente l'hydrogène, un alkyle avec 1 à 4 atomes de carbone, un alcoxy avec 1 à 4 atomes de carbone ou un halogène, ou

$R_{11}$ et $R_{12}$ sont fixés sur des atomes de carbone voisins et représentent ensemble un méthylènedioxy ou un éthylènedioxy,

$R_4$ est un des groupes a, b ou c définis précédemment,

$R_5$ représente l'hydrogène, un alkyle avec 1 à 4 atomes de carbone, un alcoxy avec 1 à 4 atomes de carbone, un hydroxy, un halogène, un alkylthio avec 1 à 4 atomes de carbone, un nitro, un trifluorométhyle, un cyano, un amino, un amino mono- ou disubstitué par un alkyle avec 1 à 4 atomes de carbone, un monoalcanoylamino avec 1 à 4 atomes de carbone, un N–$C_1$–$C_4$-alkyl–N–$C_1$–$C_4$-alcanoylamino ou un alcanoyloxy avec 1 à 4 atomes de carbone et

$R_6$ représente l'hydrogène, un alkyle avec 1 à 4 atomes de carbone, un alcoxy avec 1 à 4 atomes de carbone ou un halogène, ou $R_5$ et $R_6$ sont fixés sur des atomes de carbone voisins et représentent ensemble un méthylènedioxy ou un éthylènedioxy, ainsi que leurs isomères optiques et leurs sels d'addition avec des acides.

2. 2-acylaminométhyl -1H-2,3- dihydro -1,4-benzodiazépines selon la revendication 1, dans lesquelles $R_1$, $R_2$, $R_4$, $R_5$, et $R_6$ ont la signification indiquée précédemment et $R_3$ est le groupe d.

3. 2-acylaminométhyl -1H-2,3- dihydro -1,4-benzodiazépines selon la revendication 1, dans lesquelles $R_1$, $R_2$, $R_3$, $R_5$ et $R_6$ ont la signification indiquée précédemment et $R_4$ est le groupe a.

4. 2-acylaminométhyl -1H-2,3- dihydro -1,4-benzodiazépines selon les revendications 1 à 3, dans lesquelles $R_3$, $R_4$, $R_5$ et $R_6$ ont la signification indiquée précédemment et $R_1$ est l'hydrogène, un alkyle avec 1 à 4 atomes de carbone ou un cyclopropylméthyle, $R_2$ représente l'hydrogène ou un alkyle avec 1 à 4 atomes de carbone et n est 0 ou 1.

5. 2-acylaminométhyl -1H-2,3-dihydro -1,4-benzodiazépines selon la revendication 1, dans lesquelles:

$R_1$ représente l'hydrogène ou un alkyle avec 1 à 4 atomes de carbone,

$R_2$ représente l'hydrogène,

n possède la signification indiqueée dans la revendication 1,

$R_3$ est un groupe a dans lequel X a la signification indiquée dans la revendication 1 et $R_7$ représente l'hydrogène, un alkyle avec 1 à 4 atomes de carbone, ou dans le cas où X est du soufre, également du fluor, du chlore ou du brome, ou est un groupe b dans lequel $R_8$ et $R_9$ possèdent les significations indiquées dans la revendication 1 ou est un groupe c dans lequel $R_{10}$ représente de l'hydrogène, ou est un groupe d dans lequel $R_{11}$ représente l'hydrogène, un alkyle avec 1 à 4 atomes de carbone, un alcoxy avec 1 à 4 atomes de carbone, un halogène ou un cyano et $R_{12}$ représente l'hydrogène un alkyle avec 1 à 4 atomes de carbone, un alcoxy avec 1 à 4 atomes de carbone ou un halogène ou $R_{12}$ et $R_{11}$ sont fixés sur des atomes de carbone voisins et représentent, conjointement, un méthylènedioxy,

$R_4$ est un des groupes a, b ou c définis précédemment,

$R_5$ représente l'hydrogène, un alkyle avec 1 à 4 atomes de carbone, un alcoxy avec 1 à 4 atomes de carbone, un halogène ou un nitro,

$R_6$ représente l'hydrogène, un alkyle avec 1 à 4 atomes de carbone, un alcoxy avec 1 à 4 atomes de carbone ou un halogène ou $R_5$ et $R_6$ sont fixés sur des atomes de carbone voisins et représentent, conjointement, un méthylènedioxy.

6. 2-acylaminométhyl -1H-2,3- dihydro -1,4-benzodiazépines selon la revendication 5, dans lesquelles $R_1$, $R_2$, $R_3$, $R_4$, $R_5$ et $R_6$ ont la significa-

tion précédente et n est 0 ou, dans le cas où $R_3$ représente un groupe d, est également 1.

7. 2-acylaminométhyl -1H-2,3- dihydro -1,4-benzodiazépines selon la revendication 5 ou 6 dans lesquelles$R_1$, $R_2$, $R_3$, $R_5$, $R_6$ et n ont la signification précédente et $R_4$ est le groupe a.

8. 1-méthyl-2-[ (4-cyanobenzoyl) -aminométhyl]-5- (3'-thiényl) -1H-2,3- dihydro-1,4-benzodiazépine et ses sels d'addition avec des acides selon la revendication 1.

9. 2-aminométhyl -1H-2,3- dihydro -1,4- benzodiazépines répondant à la formule générale IIa:

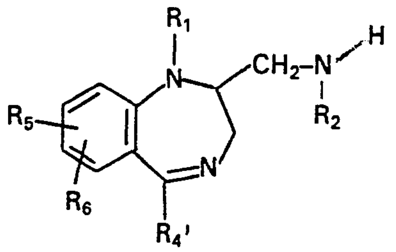

IIa

dans laquelle $R_1$, $R_2$, $R_5$ et $R_6$ possèdent la signification indiquée dans la revendication 1,

$R_4'$ est un des groupes a', b ou c suivants:

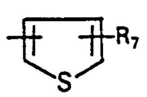

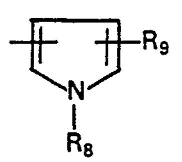

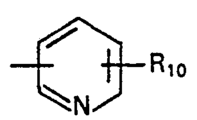

a' b c

où $R_7$, $R_8$, $R_9$ et $R_{10}$ ont la signification indiquée dans la revendication 1, ainsi que leurs isomères optiques et leurs sels d'addition avec des acides.

10. 2-azidométhyl -1H-2,3-dihydro -1,4- benzodiazépines répondant à la formule générale IV

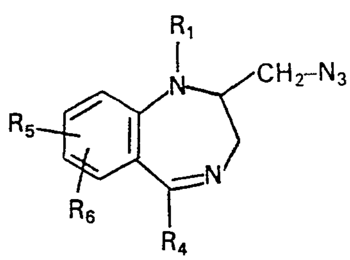

IV

dans laquelle $R_1$, $R_5$ et $R_6$ ont la signification indiquée dans la revendication 1,

$R_4$ est un des groupes a, b ou c suivants:

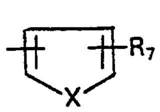

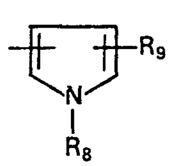

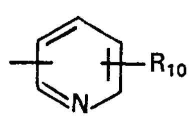

a b c

où X, $R_7$, $R_8$, $R_9$ et $R_{10}$ ont la signification indiquée dans la revendication 1, ainsi que leurs isomères optiques et leurs sels d'addition avec des acides.

11. Procédé de préparation de 2-acylaminométhyl -1H-2,3- dihydro -1,4- benzodiazépines de formule générale I:

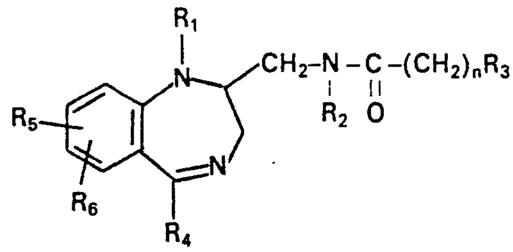

I

dans laquelle n, $R_1$, $R_2$, $R_3$, $R_4$, $R_5$ et $R_6$ ont la signification indiquée dans la revendication 1, ainsi que de leurs isomères optiques et de leurs sels d'addition avec des acides, caractérisé en ce qu'on acyle des 2-aminométhyl -1H-2,3- dihydro -1,4- benzodiazépines de formule II:

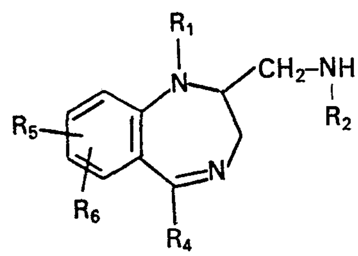

II

dans laquelle $R_1$, $R_2$, $R_4$, $R_5$ et $R_6$ ont la signification ci-dessus, avec des acides ou des dérivés d'acides réactifs de formule III:

$R_3\,(CH_2)_n\text{–CO–Y}$ III

dans laquelle $R_3$ et n ont la signification précédente et Y représente un hydroxy ou un groupe éliminable par aminolyse, qu'on alkyle le cas échéant des composés de formule I où $R_2$ représente l'hydrogène en des composés de formule I dans laquelle $R_2$ représente un alkyle avec 1 à 4 atomes de carbone, un allyle, un butényle-2 ou un buténylе-3, qu'on dédouble le cas échéant les mélanges racémiques des composés de formule I en leurs isomères optiques et transforme éventuellement des composés libres de formule I en leurs sels d'addition avec des acides ou transforme les sels d'addition avec des acides en les composés libres de formule I.

12. Produits pharmaceutiques renfermant une quantité pharmacologiquement efficace d'une 2-acylaminométhyl -1H-2,3- dihydro -1,4- benzodiazépine selon la revendication 1 et les adjuvants et/ou véhicules usuels.

**Revendications pour l'etat contractant: AT**

1. Procédé de préparation de 2-acylaminométhyl -1H-2,3- dihydro -1,4- benzodiazépines de formule générale I:

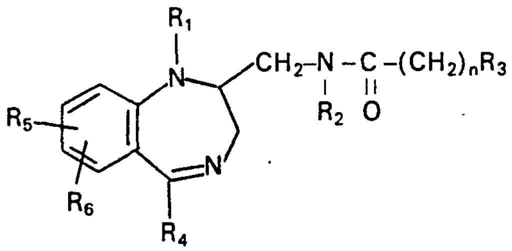 I

dans laquelle:

$R_1$ représente l'hydrogène, un alkyle avec 1 à 4 atomes de carbone, un allyle, un butényle-2, un buténvle-3 ou un cyclopropylméthyle,

$R_2$ représente l'hydrogène, un alkyle avec 1 à 4 atomes de carbone, un allyle, un butényle-2 ou un buténvle-3;

n est 0, 1 ou 2,

$R_3$ est un des groupes a, b, c ou d suivants:

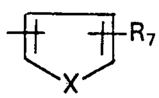

a

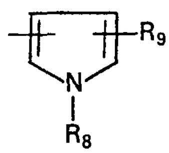

b

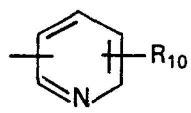

c

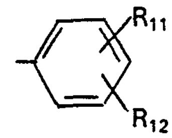

d

où

X représente de l'oxygène ou du soufre,

$R_7$ représente l'hydrogène, un alkyle avec 1 à 4 atomes de carbone, un alcoxy avec 1 à 4 atomes de carbone, un nitro ou un halogène,

$R_8$ représente l'hydrogène ou un alkyle avec 1 à 4 atomes de carbone,

$R_9$ représente l'hydrogène ou un alkyle avec 1 à 4 atomes de carbone,

$R_{10}$ représente l'hydrogène, un alkyle avec 1 à 4 atomes de carbone ou du chlore,

$R_{11}$ représente l'hydrogène, un alkyle avec 1 à 4 atomes de carbone, un alcoxy avec 1 à 4 atomes de carbone, un hydroxy, un halogène, un alkylthio avec 1 à 4 atomes de carbone, un nitro, un trifluorométhyle, un cyano, un amino, un amino mono- ou disubstitué par un alkyle avec 1 à 4 atomes de carbone, un N–$C_1$ à $C_4$-alkyl–N–$C_1$ à $C_4$-alcanoylamino ou un alcanoyloxy avec 1 à 4 atomes de carbone,

$R_{12}$ représente l'hydrogène, un alkyle avec 1 à 4 atomes de carbone, un alcoxy avec 1 à 4 atomes de carbone ou un halogène, ou

$R_{11}$ et $R_{12}$ sont fixés sur des atomes de carbone voisins et représentent ensemble un méthylènedioxy ou un éthylène-dioxy,

$R_4$ est un des groupes a, b ou c définis précédemment,

$R_5$ représente l'hydrogène, un alkyle avec 1 à 4 atomes de carbone, un alcoxy avec 1 à 4 atomes de carbone, un hydroxy, un halogène, un alkylthio avec 1 à 4 atomes de carbone, un nitro, un trifluorométhyle, un cyano, un amino, un amino mono- ou disubstitué par un alkyle avec 1 à 4 atomes de carbone, un monoalcanoylamino avec 1 à 4 atomes de carbone, un N–$C_1$–$C_4$-alkyl–N–$C_1$–$C_4$-alcanoylamino ou un alcanoyloxy avec 1 à 4 atomes de carbone et

$R_6$ représente l'hydrogène, un alkyle avec 1 à 4 atomes de carbone, un alcoxy avec 1 à 4 atomes de carbone ou un halogène, ou

$R_5$ et $R_6$ sont fixés sur des atomes de carbone voisins et représentent ensemble un méthylènedioxy ou un éthylènedioxy, ainsi que leurs isomères optiques et leurs sels d'addition avec des acides, caractérisé en ce qu'on acyle des composés de 2-aminométhyl -1H-2,3- dihydro -1,4- benzodiazépine de formule II:

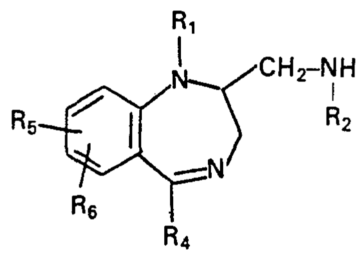 II

dans laquelle $R_1$, $R_2$, $R_4$, $R_5$ et $R_6$ ont la signification ci-dessus, avec des acides ou des dérivés d'acides réactifs de formule III:

$R_3$–$(CH_2)_n$–CO–Y III

dans laquelle

$R_3$ et n ont la signification précédente et Y représente un hydroxy ou un groupe éliminable par aminolyse, qu'on alkyle le cas échéant des composés de formule I, où $R_2$ représente l'hydrogène en des composés de formule I, dans laquelle $R_2$ représente un alkyle avec 1 à 4 atomes de carbone, un allyle, un buténvle-2 ou un buténvle-3, qu'on dédouble le cas échéant les mélanges racémiques des composés de formule I en leurs isomères optiques et transforme éventuellement des composés libres de formule I en leurs sels d'addition avec des acides ou transforme les sels d'addition avec des acides en les composés libres de formule I.

2. Procédé selon la revendication 1, caractérisé en ce qu'on prépare des 2-acylaminométhyl -1H-2,3- dihydro -1,4- benzodiazépines de formule I, dans laquelle $R_1$, $R_2$, $R_4$, $R_5$ et $R_6$ ont la signification précédente et $R_3$ est le groupe d.

3. Procédé selon la revendication 1, caractérisé en ce qu'on prépare des 2-acylaminométhyl -1H-2,3- dihydro -1,4- benzodiazépines de formule I dans laquelle $R_1$, $R_2$, $R_3$, $R_5$ et $R_6$ ont la signification précédente et $R_4$ est le groupe a.

4. Procédé selon les revendications 1 à 3, caractérisé en ce qu'on prépare des 2-acylaminométhyl -1H-2,3- dihydro -1,4- benzodiazépines de for-

mule I dans laquelle $R_3$, $R_4$, $R_5$ et $R_6$ ont la signification précédente et $R_1$ représente l'hydrogène, un alkyle avec 1 à 4 atomes de carbone ou un cyclopropylméthyle, $R_2$ représente de l'hydrogène ou un alkyle avec 1 à 4 atomes de carbone et n est 0 ou 1.

5. Procédé selon la revendication 1, caractérisé en ce qu'on prépare des 2-acylaminométhyl -1H-2,3- dihydro -1,4- benzodiazépines de formule I dans laquelle:

$R_1$ représente l'hydrogène ou un alkyle avec 1 à 4 atomes de carbone.

$R_2$ représente l'hydrogène,

n possède la signification indiquée dans la revendication 1,

$R_3$ est un groupe a dans lequel X a la signification indiquée dans la revendication 1 et $R_7$ représente l'hydrogène, un alkyle avec 1 à 4 atomes de carbone, ou dans le cas où X est du soufre, également du fluor, du chlore ou du brome, ou est un groupe b dans lequel $R_8$ et $R_9$ possèdent les significations indiquées dans la revendication 1 ou est un groupe c dans lequel $R_{10}$ représente l'hydrogène ou est un groupe d dans lequel $R_{11}$ représente l'hydrogène, un alkyle avec 1 à 4 atomes de carbone, un alcoxy avec 1 à 4 atomes carbone, un halogène ou un cyano et $R_{12}$ représente de l'hydrogène, un alkyle avec 1 à 4 atomes de carbone, un alcoxy avec 1 à 4 atomes de carbone ou un halogène ou $R_{12}$ et $R_{11}$ sont fixés sur des atomes de carbone voisins et représentent, conjointement, un méthylènedioxy,

$R_4$ est un des groupes a, b ou c définis précédemment,

$R_5$ représente l'hydrogène, un alkyle avec 1 à 4 atomes de carbone, un alcoxy avec 1 à 4 atomes de carbone, un halogène ou un nitro,

$R_6$ représente l'hydrogène, un alkyle avec 1 à 4 atomes de carbone, un alcoxy avec 1 à 4 atomes de carbone ou un halogène ou $R_5$ et $R_6$ sont fixés sur des atomes de carbone voisins et représentent, conjointement, un méthylènedioxy.

6. Procédé selon la revendication 5, caractérisé en ce qu'on prépare des 2-acylaminométhyl -1H-2,3- dihydro -1,4- benzodiazépines de formule I, dans laquelle $R_1$, $R_2$, $R_3$, $R_4$, $R_5$ et $R_6$ ont la signification précédente et n est 0 ou, dans le cas où $R_3$ représente un groupe d, n est également 1.

7. Procédé selon la revendication 5 ou 6, caractérisé en ce qu'on prépare des 2-acylaminométhyl -1H-2,3- dihydro -1,4- benzodiazépines de formule I dans laquelle $R_1$, $R_2$, $R_3$, $R_5$ et $R_6$ et n ont la signification précédente et $R_4$ est le groupe a.

8. Procédé selon la revendication 1, caractérisé en ce qu'on prépare de la 1-méthyl-2-[ (4-cyanobenzoyl) -aminométhyl]-5- (3'-thiényl) -1H-2,3-dihydro -1,4- benzodiazépine et ses sels d'addition avec des acides.

9. Procédé de préparation de 2-aminométhyl -1H-2,3- dihydro -1,4- benzodiazépines de formule générale IIa:

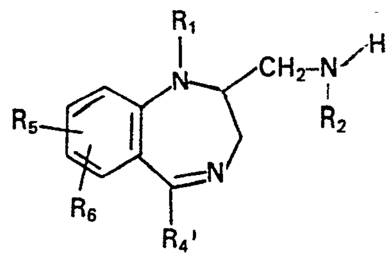

IIa

dans laquelle $R_1$, $R_2$, $R_5$ et $R_6$ possèdent la signification indiquée dans la revendication 1,

$R_{4'}$ est un des groupes a', b ou c suivants:

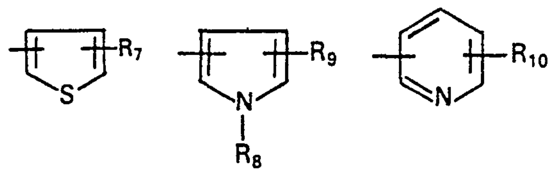

a' b c

où $R_7$, $R_8$, $R_9$ et $R_{10}$ ont la signification indiquée dans la revendication 1, ainsi que leurs isomères optiques et leurs sels d'addition avec des acides, caractérisé en ce que, dans des composés de formules IV:

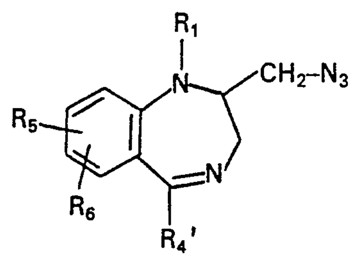

IV

dans laquelle $R_1$, $R_4'$, $R_5$ et $R_6$ ont la signification précédente, on réduit le groupe azido en groupe amino ou que, dans des composés de formule V:

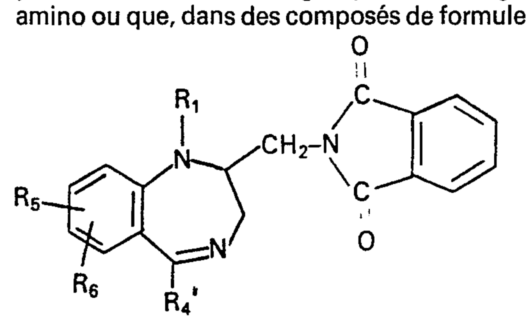

V

dans laquelle $R_1$, $R_4'$, $R_5$ et $R_6$ ont la signification ci-dessus, on hydrolyse le groupe phthalimide en groupe amino et on alkyle, le cas échéant, les composés 2-aminométhyle obtenus.

10. Procédé de préparation de 2-azidométhyl -1H-2,3-dihydro -1,4- benzodiazépines de formule générale IV

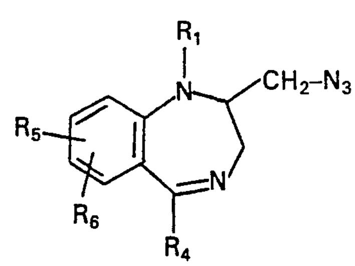

IV

dans laquelle $R_1$, $R_5$ et $R_6$ ont la signification indiquée dans la revendication 1,

$R_4$ est un des groupes a, b ou c suivants:

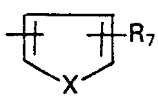
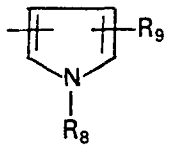
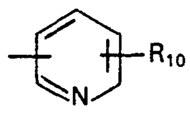

a b c

où X, $R_7$, $R_8$, $R_9$ et $R_{10}$ ont la signification indiquée dans la revendication 1, ainsi que leurs isomères optiques et leurs sels d'addition avec des acides, caractérisé en ce qu'on fait réagir des composés de formules IX et/ou des composés de formule X

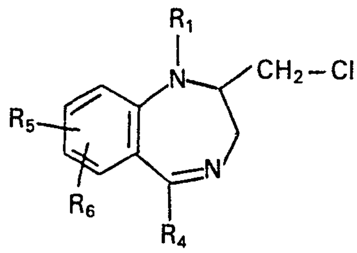

IX

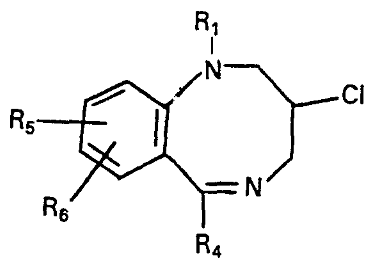

X

où $R_1$, $R_4$, $R_5$ et $R_6$ possèdent la signification ci-dessus, avec un azide de métal alcalin.

**Claims for the Contracting States: BE, CH, DE, FR, GB, IT, LI, LU, NL, SE**

1. 2-Acylaminomethyl -1H-2,3- dihydro -1,4-benzodiazepine compounds of the general formula I

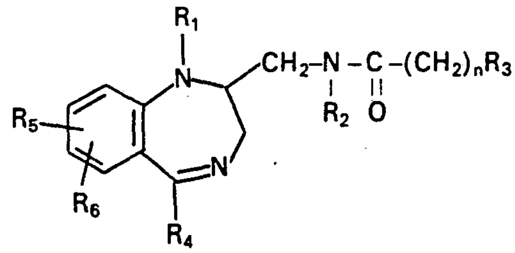

I

wherein

$R_1$ is hydrogen, alkyl with 1–4 carbon atoms, allyl, 2-butenyl, 3-butenyl or cyclopropylmethyl,

$R_2$ is hydrogen, alkyl with 1–4 carbon atoms, allyl, 2-butenyl or 3-butenyl,

n stands for 0, 1 or 2,

$R_3$ stands for one of the following groups a, b, c or d

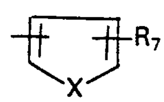
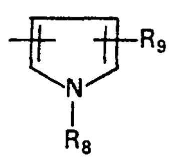
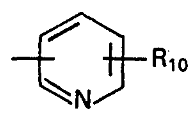
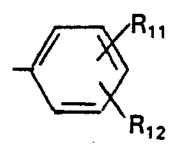

a b c d

where

X is oxygen or sulphur

$R_7$ is hydrogen, alkyl with 1–4 carbon atoms, alkoxy with 1–4 carbon atoms, nitro or halogen,

$R_8$ is hydrogen or alkyl with 1–4 carbon atoms,

$R_9$ is hydrogen or alkyl with 1–4 carbon atoms,

$R_{10}$ is hydrogen, alkyl with 1–4 carbon atoms or chlorine,

$R_{11}$ is hydrogen, alkyl with 1–4 carbon atoms, alkoxy with 1–4 carbon atoms, hydroxy, halogen, alkylthio with 1–4 carbon atoms, nitro trifluoromethyl, cyano, amino, amono mono- or di-substituted by alkyl with 1–4 carbon atoms, monoalkanoylamino with 1–4 carbon atoms, N–$C_1$–$C_4$-alkyl–N–$C_1C_4$-alkanoylamino or alkanoyloxy with 1–4 carbon atoms.

$R_{12}$ is hydrogen, alkyl with 1–4 carbon atoms, alkoxy with 1–4 carbon atoms or halogen, or

$R_{11}$ and $R_{12}$ are bonded to adjacent carbon atoms and together denote methylenedioxy or ethylenedioxy,

$R_4$ stands for one of the groups a, b or c defined above,

$R_5$ is hydrogen, alkyl with 1–4 carbon atoms, alkoxy with 1–4 carbon atoms, hydroxy, halogen, alkylthio with 1–4 carbon atoms, nitro, trifluoromethyl, cyano, amino, amino mono- or di-substituted by alkyl with 1–4 carbon atoms, monoalkanoylamino with 1–4 carbon atoms, N–$C_1$–$C_4$-alkyl–N–$C_1$–$C_4$-alkanoylamino or alkanoyloxy with 1–4 carbon atoms and

$R_6$ is hydrogen, alkyl with 1–4 carbon atoms, alkoxy with 1–4 carbon atoms or halogen, or

$R_5$ and $R_6$ are bonded to adjacent carbon atoms and together denote methylenedioxy or ethylenedioxy, and their optical isomers and their acid addition salts.

2. 2-Acylaminomethyl -1H-2,3- dihydro -1,4-benzodiazepine compounds according to Claim 1, wherein $R_1$, $R_2$, $R_4$, $R_5$ and $R_6$ have the above meaning and $R_3$ stands for the group d.

3. 2-Acylaminomethyl -1H-2,3- dihydro -1,4-benzodiazepine compounds according to Claim 1, wherein $R_1$, $R_2$, $R_3$, $R_5$ and $R_6$ have the above meaning and $R_4$ stands for the group a.

4. 2-Acylaminomethyl -1H-2,3- dihydro -1,4- benzodiazepine compounds according to Claims 1–3, wherein $R_3$, $R_4$, $R_5$ and $R_6$ have the above meaning and $R_1$ is hydrogen, alkyl with 1–4 carbon atoms or cyclopropylmethyl, $R_2$ is hydrogen or alkyl with 1–4 carbon atoms and n stands for 0 or 1.

5. 2-Acylaminomethyl -1H-2,3- dihydro -1,4- benzodiazepine compounds according to Claim 1, wherein

$R_1$ is hydrogen or alkyl with 1–4 carbon atoms,

$R_2$ is hydrogen

n has the meaning given in Claim 1,

$R_3$ stands for a group a, where X has the meaning given in Claim 1 and $R_7$ is hydrogen, alkyl with 1–4 carbon atoms or, if X is sulphur, also fluorine, chlorine or bromine, or stands for a group b, where $R_8$ and $R_9$ have the meanings given in Claim 1, or stands for a group c, where $R_{10}$ is hydrogen, or stands for a group d, where $R_{11}$ is hydrogen, alkyl with 1–4 carbon atoms, alkoxy with 1–4 carbon atoms, halogen or cyano and $R_{12}$ is hydrogen, alkyl with 1–4 carbon atoms, alkoxy with 1–4 carbon atoms, halogen or cyano and $R_{12}$ is hydrogen, alkyl with 1–4 carbon atoms, alkoxy with 1–4 carbon atoms or halogen, or $R_{12}$ and $R_{11}$ are bonded to adjacent carbon atoms and together denote methylenedioxy,

$R_4$ stands for one of the groups a, b or c defined above,

$R_5$ is hydrogen, alkyl with 1–4 carbon atoms, alkoxy with 1–4 carbon atoms, halogen or nitro,

$R_6$ is hydrogen, alkyl with 1–4 carbon atoms, alkoxy with 1–4 carbon atoms or halogen or

$R_5$ and $R_6$ are bonded to adjacent carbon atoms and together denote methylenedioxy.

6. 2-Acylaminomethyl -1H-2,3- dihydro -1,4- benzodiazepine compounds according to Claim 5, wherein $R_1$, $R_2$, $R_3$, $R_4$, $R_5$ and $R_6$ have the above meaning and n stands for 0 or, if $R_3$ is a group d, also stands for 1.

7. 2-Acylaminomethyl -1H-2,3- dihydro -1,4- benzodiazepine compounds according to Claim 5 or 6, wherein $R_1$, $R_2$, $R_3$, $R_5$, $R_6$ and n have the above meaning and $R_4$ stands for a group a.

8. 1-Methyl-2- [(4-cyanobenzoyl) -aminomethyl] -5- (3'-thienyl) -1H-2,3-dihydro-1,4-benzodiazepine and its acid addition salts according to Claim 1.

9. 2-Aminomethyl -1H-2,3- dihydro -1,4- benzodiazepine compounds of the general formula IIa

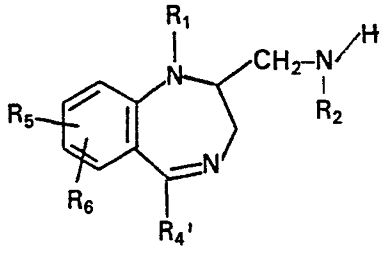

IIa

wherein $R_1$, $R_2$, $R_5$ and $R_6$ have the meaning indicated in Claim 1, $R_4$ stands for one of the following groups a', b or c

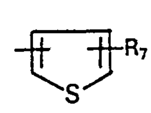

a'

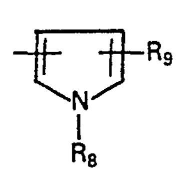

b

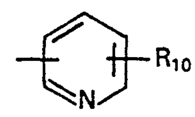

c

where $R_7$, $R_8$, $R_9$ and $R_{10}$ have the meaning indicated in Claim 1, and their optical isomers and their acid addition salts.

10. 2-Azidomethyl -1H-2,3- dihydro -1,4- benzodiazepine compounds of the general formula IV

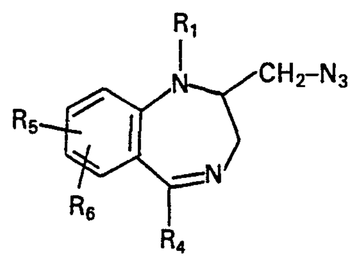

IV

wherein $R_1$, $R_5$ and $R_6$ have the meaning indicated in Claim 1, $R_4$ stands for one of the following groups a, b or c

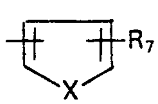

a

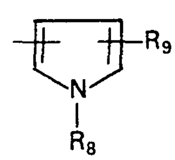

b

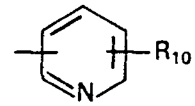

c

where X, $R_7$, $R_8$, $R_9$ and $R_{10}$ have the meaning indicated in Claim 1, and their optical isomers and their acid addition salts.

11. Process for the preparation of 2-acylaminomethyl -1H-2,3- dihydro -1,4- benzodiazepine compounds of the general formula I

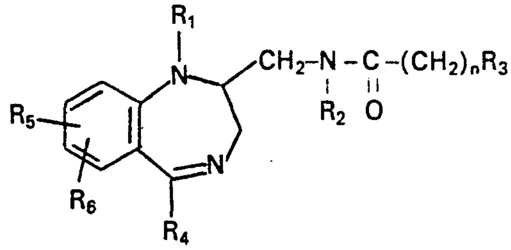

I

wherein n, $R_1$, $R_2$, $R_3$, $R_4$, $R_5$ and $R_6$ have the meaning indicated in Claim 1, and their optical isomers and their acid addition salts, characterized in that 2-aminomethyl -1H-2,3- dihydro -1,4- benzodiazepine compounds of the formula II

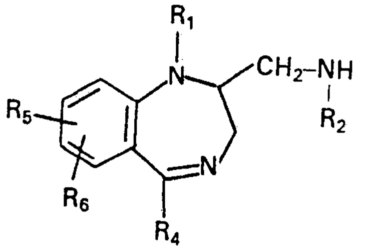

II

wherein $R_1$, $R_2$, $R_4$, $R_5$ and $R_6$ have the above meaning are acylated with acids or reactive acid derivatives of the formula III

$R_3-(CH_2)_n-CO-Y$ III

wherein $R_3$ and n have the above meaning and Y is hydroxy or a group which can be split off aminolytically, and, if necessary, compounds of the formula I, wherein $R_2$ denotes hydrogen are alkylated to compounds of the formula I wherein $R_2$ denotes alkyl with 1–4 carbon atoms, allyl, 2-butenyl or 3-butenyl, and if required racemic mixtures of compounds of the formula I are separated into their optical isomers and, if required, free compounds of the formula I are converted into their acid addition salts or the acid addition salts are converted into the free compounds of the formula I.

12. Medicament containing a pharmacologically effective quantity of a 2-acylaminomethyl -1H-2,3- dihydro -1,4- benzodiazepine compound according to Claim 1 and conventional adjuvant and/or carrier substances.

**Claims for the Contracting State: Austria**

1. Process for the preparation of 2-acylaminomethyl -1H-2,3- dihydro -1,4- benzodiazepine compounds of the general formula I

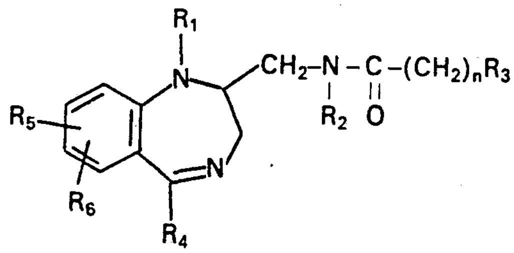

I

wherein

$R_1$ is hydrogen, alkyl with 1–4 carbon atoms, allyl, 2-butenyl, 3-butenyl or cyclopropylmethyl,

$R_2$ is hydrogen, alkyl with 1–4 carbon atoms, allyl, 2-butenyl or 3-butenyl,

n stands for 0, 1 or 2,

$R_3$ stands for one of the following groups a, b, c or d

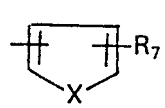

a

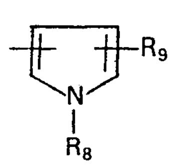

b

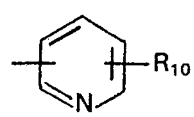

c

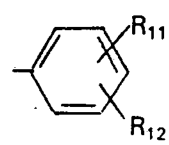

d

where

X is oxygen or sulphur

$R_7$ is hydrogen, alkyl with 1–4 carbon atoms, alkoxy with 1–4 carbon atoms, nitro or halogen,

$R_8$ is hydrogen or alkyl with 1-4 carbon atoms,

$R_9$ is hydrogen or alkyl with 1–4 carbon atoms,

$R_{10}$ is hydrogen, alkyl with 1–4 carbon atoms or chlorine,

$R_{11}$ is hydrogen, alkyl with 1–4 carbon atoms, alkoxy with 1–4 carbon atoms, hydroxy, halogen, alkylthio with 1–4 carbon atoms, nitro, trifluoromethyl, cyano, amino, amino mono- or di-substituted by alkyl with 1–4 carbon atoms, monoalkanoylamino with 1–4 carbon atoms, $N-C_1-C_4$-alkyl-$N-C_1-C_4$-alkanoylamino or alkanoyloxy with 1–4 carbon atoms.

$R_{12}$ is hydrogen, alkyl with 1–4 carbon atoms, alkoxy with 1–4 carbon atoms or halogen, or

$R_{11}$ and $R_{12}$ are bonded to adjacent carbon atoms and together denote methylenedioxy or ethylenedioxy,

$R_4$ stands for one of the groups a, b or c defined above,

$R_5$ is hydrogen, alkyl with 1–4 carbon atoms, alkoxy with 1–4 carbon atoms, hydroxy, halogen, alkylthio with 1–4 carbon atoms, nitro, trifluoromethyl, cyano, amino, amino mono- or di-substituted by alkyl with 1–4 carbon atoms, monoalkanoylamino with 1–4 carbon atoms, $N-C_1-C_4$-alkyl-$N-C_1-C_4$-alkanoylamino or alkanoyloxy with 1–4 carbon atoms and

$R_6$ is hydrogen, alkyl with 1–4 carbon atoms, alkoxy with 1–4 carbon atoms or halogen, or

$R_5$ and $R_6$ are bonded to adjacent carbon atoms and together denote methylenedioxy or ethylenedioxy, and their optical isomers and their acid addition salts, characterized in that 2-aminomethyl -1H-2,3- dihydro -1,4- benzodiazepine compounds of the formula II

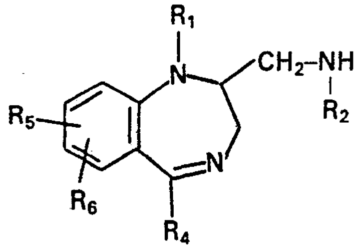

II

wherein $R_1$, $R_2$, $R_4$, $R_5$ and $R_6$ have the above meaning are acylated with acids or reactive acid derivatives of the formula III

$R_3-(CH_2)_n-CO-Y$ III

wherein $R_3$ and n have the above meaning and Y is hydroxy or a group which can be split off aminolytically, and, if necessary, compounds of the formula I, wherein $R_2$ denotes hydrogen are alkylated to compounds of the formula I wherein $R_2$ denotes alkyl with 1–4 carbon atoms, allyl, 2-butenyl or 3-butenyl, and if required racemic mixtures of compounds of the formula I are separated into their optical isomers and, if required, free compounds of the formula I are converted into their acid addition salts or the acid addition salts are converted into the free compounds of the formula I.

2. Process according to Claim 1, characterized in that 2-acylaminomethyl -1H-2,3- dihydro -1,4- benzodiazepine compounds of formula I are prepared, wherein $R_1$, $R_2$, $R_4$, $R_5$ and $R_6$ have the above meaning and $R_3$ stands for the group d.

3. Process according to Claim 1, characterized in that 2-acylaminomethyl -1H-2,3- dihydro -1,4- benzodiazepine compounds of formula I are prepared, wherein $R_1$, $R_2$, $R_3$, $R_5$ and $R_6$ have the above meaning and $R_4$ stands for the group a.

4. Process according to Claims 1 to 3, characterized in that 2-acylaminomethyl-1H-2,3- dihydro -1,4- benzodiazepine compounds of formula I are prepared, wherein $R_3$, $R_4$, $R_5$ and $R_6$ have the above meaning and $R_1$ is hydrogen, alkyl with 1–4 carbon atoms or cyclopropylmethyl, $R_2$ is hydrogen or alkyl with 1–4 carbon atoms and n stands for 0 or 1.

5. Process according to Claim 1, characterized in that 2-acylaminomethyl -1H-2,3-dihydro -1,4- benzodiazepine componds of formula I are prepared, wherein

$R_1$ is hydrogen or alkyl with 1–4 carbon atoms,

$R_2$ is hydrogen,

n has the meaning given in Claim 1,

$R_3$ stands for a group a, where X has the meaning indicated in Claim 1 and $R_7$ is hydrogen, alkyl with 1–4 carbon atoms or, if X is sulphur, also fluorine, chlorine or bromine, or stands for a group b, where $R_8$ and $R_9$ have the meanings indicated in Claim 1, or stands for a group c, in which $R_{10}$ is hydrogen, or stands for a group d, in which $R_{11}$ is hydrogen, alkyl with 1–4 carbon atoms, alkoxy with 1–4 carbon atoms, halogen or cyano and $R_{12}$ is hydrogen, alkyl with 1–4 carbon atoms, alkoxy with 1–4 carbon atoms or halogen, or $R_{12}$ and $R_{11}$ are bonded to adjacent carbon atoms and together denote methylenedioxy,

$R_4$ stands for one of the groups a, b or c defined above,

$R_5$ is hydrogen, alkyl with 1–4 carbon atoms, alkoxy with 1–4 carbon atoms, halogen or nitro,

$R_6$ is hydrogen, alkyl with 1–4 carbon atoms, alkoxy with 1–4 carbon atoms or halogen or

$R_5$ and $R_6$ are bonded to adjacent carbon atoms and together denote methylenedioxy.

6. Process according to Claim 5, characterized in that 2-acylaminomethyl -1H-2,3- dihydro -1,4- benzodiazepine compounds of formula I are prepared, wherein $R_1$, $R_2$, $R_3$, $R_4$, $R_5$ and $R_6$ have the above meaning and n stands for 0 or, if $R_3$ is a group d, also stands for 1.

7. Process according to Claim 5 or 6, characterized in that 2-acylaminomethyl -1H-2,3- dihydro -1,4- benzodiazepine compounds of formula I are prepared, wherein $R_1$, $R_2$, $R_3$, $R_5$, $R_6$ and n have the above meaning and $R_4$ stands for the group a.

8. Pocess according to Claim 1, characterized in that 1-methyl-2-[(4-cyanobenzoyl) -amino-methyl] -5- (3'-thienyl) -1H-2,3- dihydro-1,4-benzodiazepine and its acid addition salts are prepared.

9. Process for the preparation of 2-aminomethyl-1H-2,3- dihydro -1,4- benzodiazepine compounds of the general formula IIa

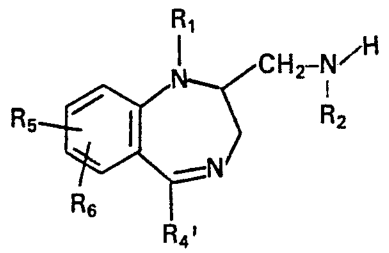

IIa

wherein $R_1$, $R_2$, $R_5$ and $R_6$ have the meaning indicated in Claim 1, $R_4'$ stands for one of the following groups a', b or c

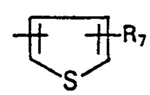 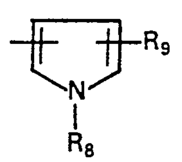 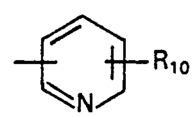

a' b c

wherein $R_7$, $R_8$, $R_9$ and $R_{10}$ have the meaning indicated in Claim 1, and their optical isomers and their acid addition salts, characterized in that in compounds of the general formula IV

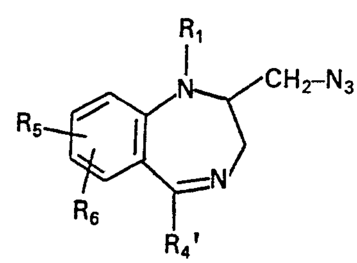

IV

wherein $R_1$, $R_4'$, $R_5$ and $R_6$ have the above meaning, the azido group is reduced to the amino group or is hydrolysed in compounds of formula V

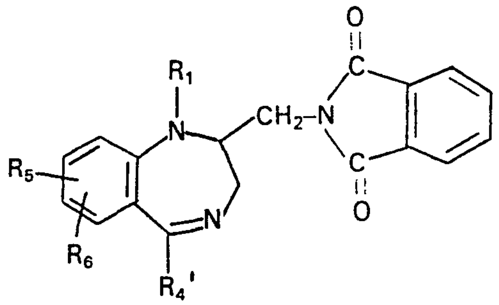

V

wherein $R_1$, $R_4'$, $R_5$ and $R_6$ have the above meaning, the phthalimide group is hydrolysed to the amino group and if required the 2-aminomethyl compounds abtained are alkylated.

10. Process for the preparation of 2-azidomethyl -1H-2,3- dihydro -1,4- benzodiazepine compounds of the general formule IV

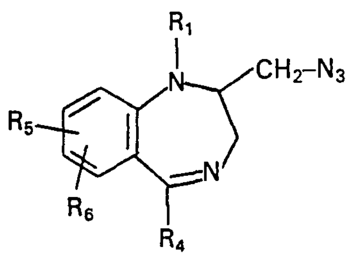

IV

wherein $R_1$, $R_5$ and $R_6$ have the meaning indicated in Claim 1, $R_4$ stands for one of the following groups a, b or c

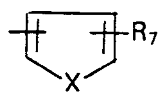

a

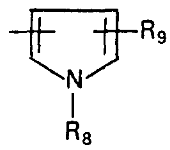

b

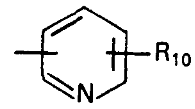

c

wherein X, $R_7$, $R_8$, $R_9$ and $R_{10}$ have the meaning indicated in Claim 1, and their optical isomers and their acid addition salts, characterized in that compounds of formula IX and/or compounds of formula X

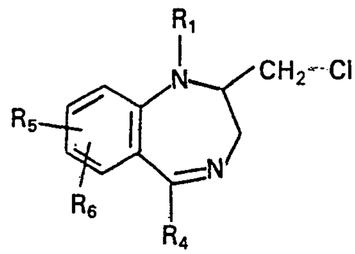

IX

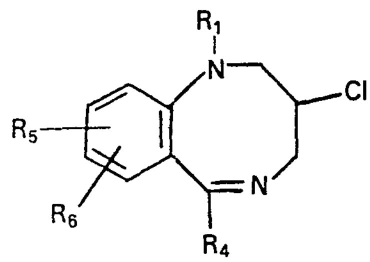

X

wherein $R_1$, $R_4$, $R_5$ and $R_6$ have the above meaning, are reacted with an alkali metal azide.